# EUROPÄISCHE PATENTSCHRIFT

(11) **EP 3 113 618 B1**
(45) Veröffentlichungstag und Bekanntmachung des Hinweises auf die Patenterteilung: **04.07.2018**
(21) Anmeldenummer: 15710136.1
(22) Anmeldetag: 04.03.2015
(51) Int. Cl.: A01N 43/653, A01N 43/713, C07D 401/04, C07D 249/14, C07D 257/06, A01P 5/00, A01P 7/00, A01P 9/00

(54) **HETEROCYCLISCHE VERBINDUNGEN ALS SCHÄDLINGSBEKÄMPFUNGSMITTEL**
HETEROCYCLIC COMPOUNDS AS PEST CONTROLLERS
COMPOSÉS HÉTÉROCYCLIQUES EN TANT QUE MOYEN DE LUTTE CONTRE LES PARASITES

(30) Priorität: 06.03.2014 EP 14157977
(43) Veröffentlichungstag der Anmeldung: 11.01.2017
(73) Patentinhaber: Bayer CropScience Aktiengesellschaft, 40789 Monheim am Rhein (DE)
(72) Erfinder: HEILMANN, Eike Kevin, 40229 Düsseldorf (DE); WROBLOWSKY, Heinz-Juergen, 40764 Langenfeld (DE); TRAUTWEIN, Axel, 40593 Duesseldorf (DE); GREUL, Jörg, 51381 Leverkusen (DE); DEMBSKI, Hardwin, 40699 Erkrath (DE); ILG, Kerstin, 50670 Köln (DE); PORTZ, Daniela, 52391 Vettweiss (DE); GÖRGENS, Ulrich, 40882 Ratingen (DE)
(74) Vertreter: BIP Patents
(86) Internationale Anmeldenummer: PCT/EP2015/054550
(87) Internationale Veröffentlichungsnummer: WO 2015/132313

(56) Entgegenhaltungen:
- EP-A1- 0 957 094
- WO-A1-2012/052412
- WO-A2-2012/116246
- DATABASE CA [Online] CHEMICAL ABSTRACTS SERVICE, COLUMBUS, OHIO, US; ZHANG, HAI-HAO ET AL: "Synthesis and Characterization of 3-amino (nitro)-5-nitro-1, 2, 4-triazole derivatives", XP002726710, gefunden im STN Database accession no. 155:96525 & ZHANG, HAI-HAO ET AL: "Synthesis and Characterization of 3-amino (nitro)-5-nitro-1, 2, 4-triazole derivatives", HANNENG CAILIAO , 18(1), 29-33 CODEN: HACAFQ; ISSN: 1006-9941, 2010, DOI: 10.3969/J.ISSN.1006-9941.2010.01.008 10.3969/J.ISSN.1006-9941.2010.01.008
- DATABASE CA [Online] CHEMICAL ABSTRACTS SERVICE, COLUMBUS, OHIO, US; TRINKA, PETER ET AL: "Triazoles. XXXVI. The arylation of 5-amino-3-(methylthio)-1H-1,2,4-triazole with activated aryl chlorides", XP002726711, gefunden im STN Database accession no. 124:8707 & TRINKA, PETER ET AL: "Triazoles. XXXVI. The arylation of 5-amino-3-(methylthio)-1H-1,2,4-triazole with activated aryl chlorides", JOURNAL OF HETEROCYCLIC CHEMISTRY , 32(4), 1359-71 CODEN: JHTCAD; ISSN: 0022-152X, 1995, DOI: 10.1002/JHET.5570320443 10.1002/JHET.5570320443
- DATABASE CA [Online] CHEMICAL ABSTRACTS SERVICE, COLUMBUS, OHIO, US; REITER, JOZSEF ET AL: "1,2,4-Triazole derivatives", XP002726712, gefunden im STN Database accession no. 100:79835 & HU 25 867 A2 (GYOGYSZERKUTATO INTEZET, HUNG.) 29. August 1983 (1983-08-29)

## Beschreibung

Die vorliegende Anmeldung betrifft neue heterocyclische Verbindungen, ihre Verwendung zur Bekämpfung von tierischen Schädlingen, zu denen Arthropoden, Insekten und Nematoden zählen, sowie Verfahren und Zwischenprodukte zur Herstellung der neuen Verbindungen.

WO 2012/116246 A2 und WO 2012/052412 A1 offenbaren Schädlingsbekämpfungsmittel auf Basis heterozyklischer Verbindungen, deren Strukturen sich jedoch von den hier beanspruchten Verbindungen unterscheiden.

Derivate von 1-Aryl-3-aroylamino-1,2,4-triazolen sind aus WO 2012/006473 bekannt.

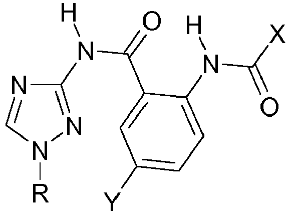

Die dort beschriebenen Verbindungen weisen Aktivität als Phosphata inhibitoren auf.

Ferner sind die folgenden Verbindungen in Scifinder als "Commercial Source" bekannt (CAS-Nr. der 2-Cl-Verbindung = 321434-03-1, der 4-Cl-Verbindung = 303144-57-2), ohne dass eine Verwendung beschrieben worden wäre:

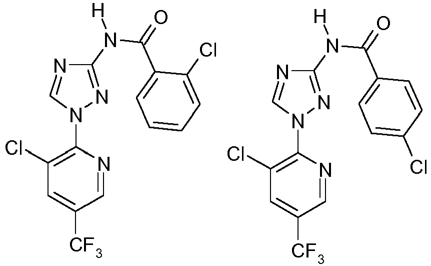

Pflanzenschutzmittel, zu denen auch Schädlingsbekämpfungsmittel gehören, müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch Verbindungen der Formel (I) 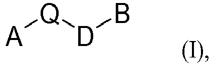 worin
- A: für einen Rest aus der Reihe 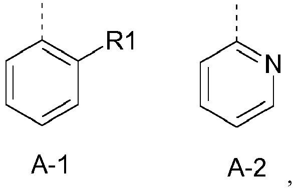 steht, in dem die gestrichelte Linie die Bindung zu Q bedeutet und wobei A weiterhin m Substituenten R2 trägt,
- Q: für einen Rest aus der Reihe 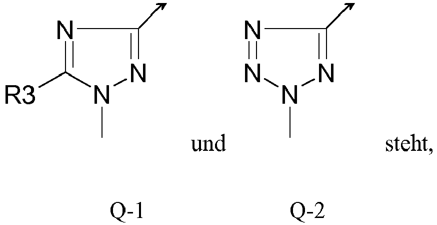 worin der Stickstoff mit dem Ring A verknüpft ist und der Pfeil jeweils die Bindung zu D bedeutet,
- D: für den Rest der Formel 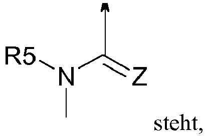 worin der Stickstoff mit Q verbunden ist und der Pfeil die Bindung zu B bedeutet,
- B: für einen Rest aus der Reihe 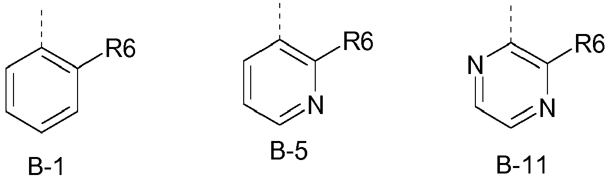 steht, worin die gestrichelte Linie die Bindung zu D bedeutet und wobei B weiterhin n Substituenten R7 trägt,
- Z: für Sauerstoff steht,
- R1: für einen Rest aus der Reihe Halogen, C₁-C₄-Alkyl, C₁-C₄-Halogenalkyl, C₁-C₄-Alkoxy und C₁-C₄-Halogenalkoxy steht,
- R2: für einen Rest aus der Reihe Halogen, C₁-C₄-Alkyl, C₁-C₄-Halogenalkyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl und C₁-C₆-Halogenalkylsulfonyl steht,
- R3: für Wasserstoff steht,
- R5: für Wasserstoff steht,
- R6: für einen Rest aus der Reihe Halogen, Nitro, C₁-C₄-Alkyl, C₁-C₄-Halogenalkyl und C₁-C₄-Halogenalkoxy steht,
- R7: für Halogen steht,
- m: für eine Zahl aus der Reihe 0, 1, 2, 3 und 4 steht, wobei für m > 1 die Reste R2 gleich oder verschieden sein können, und
- n: für eine Zahl aus der Reihe 0 und 1 steht.

Es wurde gefunden, dass die Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz, in der Tiergesundheit sowie auf dem Hygienesektor vorkommen, geeignet sind.

Durch Halogen substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die maximal möglichen Werte von m und n sind naturgemäß abhängig von der Zahl der Bindungsstellen in den Resten A und B.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Die aufgeführten Restedefinitionen bzw. Erläuterungen gelten für oben und weiter unten genannten Endprodukte, Ausgangsprodukte und Zwischenprodukte entsprechend.

In den folgenden Gruppen von Verbindungen haben die Reste R1 bis R7 und Z die oben angegebenen Bedeutungen.

In einer hervorgehobenen Gruppe von erfindungsgemäßen Verbindungen der Formel (I) steht Q für Q-1 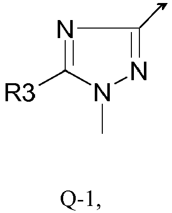 damit erhält man unter Berücksichtigung der Bedeutung von D Verbindungen der Formel (I-1)

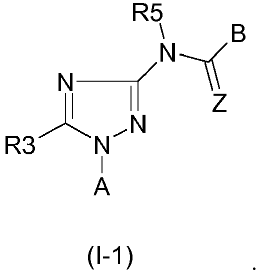

In einer weiteren hervorgehobenen Gruppe von erfindungsgemäßen Verbindungen der Formel (I) steht Q für Q-2 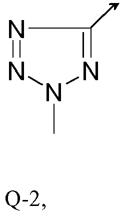 damit erhält man unter Berücksichtigung der Bedeutung von D Verbindungen der Formel (I-2)

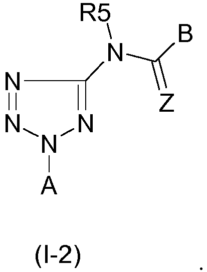

In einer weiteren hervorgehobenen Gruppe von erfindungsgemäßen Verbindungen der Formel (I) steht A für A-1 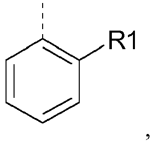 wobei dieser Rest m Substituenten R2 trägt.

In einer weiteren hervorgehobenen Gruppe von erfindungsgemäßen Verbindungen der Formel (I) steht A für A-2 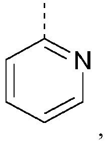 wobei dieser Rest m Substituenten R2 trägt.

In den folgenden hervorgehobenen Gruppen (G-1) bis (G-8) von Verbindungen der Formel (I), die jeweils eine bevorzugte Ausführungsform darstellen, können die Reste A und B wie oben ausgeführt weitere Substituenten R2 bzw. R7 tragen.

Eine hervorgehobene Gruppe G-1 von Verbindungen der Formel (I) sind Verbindungen der Formel (A-1)-(Q-1)-D-(B-1)

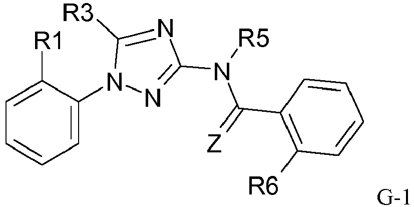

Eine weitere hervorgehobene Gruppe G-2 von Verbindungen der Formel (I) sind Verbindungen der Formel (A-2)-(Q-1)-D-(B-1)

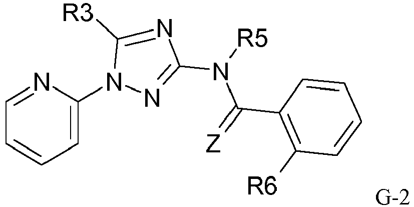

Eine weitere hervorgehobene Gruppe G-3 von Verbindungen der Formel (I) sind Verbindungen der Formel (A-1)-(Q-1)-D-(B-5)

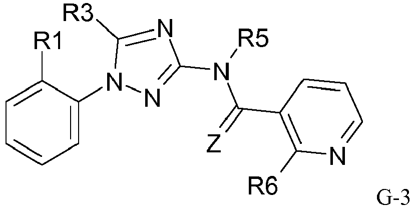

Eine weitere hervorgehobene Gruppe G-4 von Verbindungen der Formel (I) sind Verbindungen der Formel (A-1)-(Q-1)-D-(B-11)

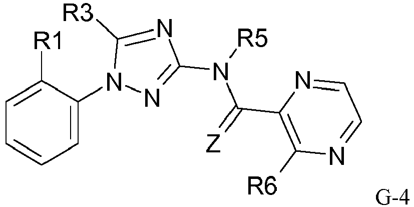

Eine weitere hervorgehobene Gruppe G-5 von Verbindungen der Formel (I) sind Verbindungen der Formel (A-1)-(Q-2)-D-(B-1)

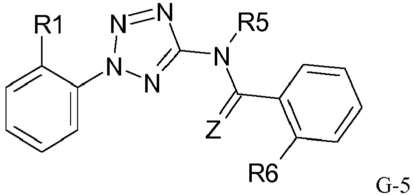

Eine weitere hervorgehobene Gruppe G-6 von Verbindungen der Formel (I) sind Verbindungen der Formel (A-2)-(Q-2)-D-(B-1)

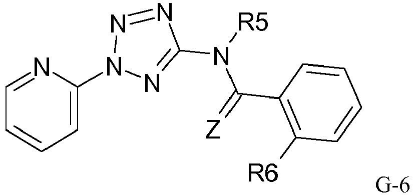

Eine weitere hervorgehobene Gruppe G-7 von Verbindungen der Formel (I) sind Verbindungen der Formel (A-1)-(Q-2)-D-(B-5)

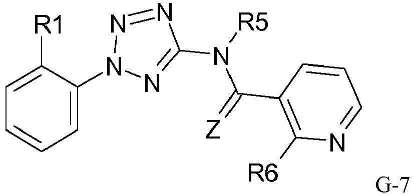

Eine weitere hervorgehobene Gruppe G-8 von Verbindungen der Formel (I) sind Verbindungen der Formel (A-1)-(Q-2)-D-(B-11)

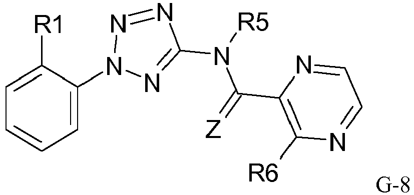

Weiter wurde gefunden, dass man die Verbindungen der Formel (I) nach den im Folgenden beschriebenen Verfahren erhalten kann. Offenbart sind auch Verfahren zur Herstellung von Verbindungen der Formel (I), in denen Z für O steht, durch Umsetzung von Aminen der Formeln (II-1) und (III-2) 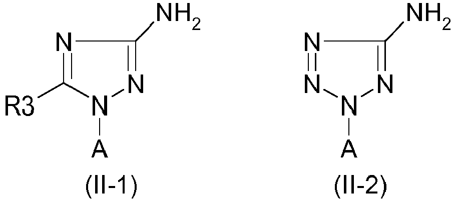 mit Verbindungen der Formel (III) 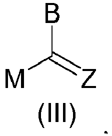 worin
- M: für Halogen, Alkoxy, Alkylsufanyl, Acyloxy, Sulfonyloxy, N-Heterocyclyl (z.B. Imidazolyl) oder für Hydroxy steht,
- B: die oben angegebenen Bedeutungen hat und
- Z: für O steht.

Hierbei können Verbindungen der Formel (III) bereits aktiviert sein oder *in situ* aktiviert werden. Beispielsweise können Verbindungen der Formel (III) als Säurehalogenide (z.B. M = Chlor) eingesetzt werden. Die Umsetzung wird dann vorteilhaft in Gegenwart einer Base, wie z.B. Triethylamin oder Natriumhydroxid durchgeführt. Es können aber auch Carbonsäuren (M = OH) in Gegenwart von Kupplungsreagenzien wie z.B. Dicyclohexylcarbodiimid und Additiven wie 1-Hydroxy-1-H-benzotriazol eingesetzt werden (W. König, R. Geiger, Chem. Ber. 1970, 103, 788). Verwendbar sind ferner Kupplungsreagenzien wie 1-Ethyl-3-(3-dimethylaminopropyl)carbodiimid, 1,1'-Carbonyl-1H-imidazol, O-(Benzotriazol-1-yl)-N,N,N',N'-tetramethyluroniumhexafluorophosphat und ähnliche Verbindungen. Als Kupplungsreagenzien zur Durchführung des Herstellungsverfahrens sind grundsätzlich alle Verbindungen geeignet, die die Bildung einer Amidbindung ermöglichen (vgl. z. B. E. Valeur, M. Bradley Chem. Soc. Rev. 2009, 38, 606; S.-Y. Han, Y.-A. Kim Tetrahedron 2004, 60, 2447). Weiterhin können auch symmetrische oder gemischte Anhydride zur Darstellung von Verbindungen der Formel (I) verwendet werden (G. W. Anderson, J. E. Zimmerman, F. M. Calahan, J. Am. Chem. Soc. 1967, 89, 5012.). Dabei können verschiedene Chlorameisensäureester zum Einsatz kommen, wie z.B. Chlorameisensäure*iso*butylester und Chlorameisensäure-*sec*-butylester. Ebenfalls können beispielsweise Isovalerylchlorid und Pivaloylchlorid verwendet werden.

Amine der Formel (II-1) 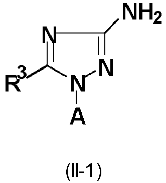 sind kommerziell erhältlich, literaturbekannt oder können anhand literaturbekannter Verfahren synthetisiert werden. Siehe z.B. WO 2012/006473, US 6083965 (2000), Anales de Quimica 70,986 (1974), J. Het. Chem. 8, 137-139 (1971), US 2010/120874, Gazz. Chimica Italiana 29, 105 (1899), J. Chem. Soc. Perkin Trans 1, 81-85 (1974), Arch. Pharm. 322, 583-587 (1989), Synthesis 45, 1093 (2013).

Neu und auch Gegenstand der Erfindung sind Verbindungen der Formel (IIa-1-a) 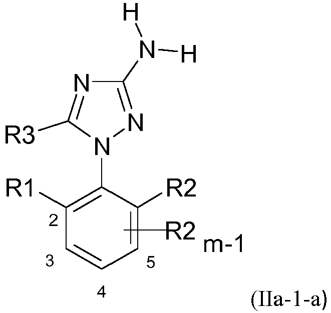 in welchen R1 und R2 die oben genannten Bedeutungen haben und m für eine Zahl aus der Reihe 1, 2 und 3 steht.

Verbindungen der Formel (I-1) lassen sich auch durch Umlagerung von N-(5-Aryl-1,2,4-oxadiazol-3-yl)-N'-arylformamidinen herstellen gemäß

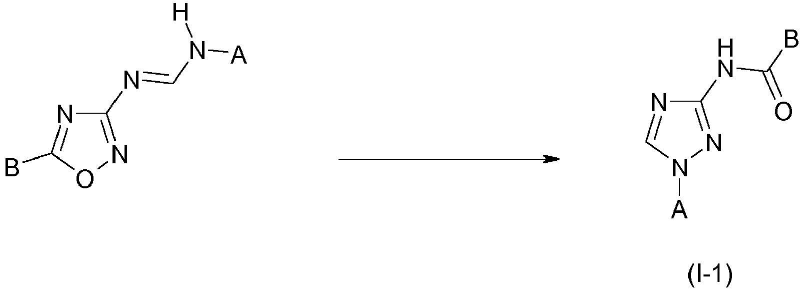

Siehe z.B. J. Chem. Soc. Perkin Trans. 2 1339-1343 (1993), Tetrahedron 50, 7315-7326 (1994), J. Het. Chem. 8, 137-139 (1971), K. H. Baumann US 2010/120874.

Man erhält die Verbindungen der Formel (I-1) auch durch Umsetzung von Alkyl-[5-aryl-1,2,4-oxadiazol-3-yl]imidoformiaten mit entsprechend substituierten Anilinen (vgl. J. Het. Chem. 8, 137-139 (1971)):

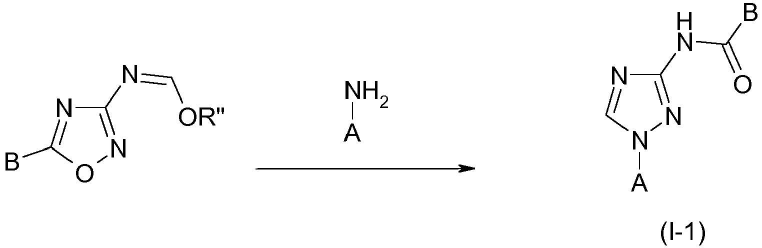

Man erhält die Verbindungen der Formel (I-1) auch durch Umlagerung von N-(4-Aryl-1,2,5-oxadiazol-3-yl)-N'-arylformamidinen (vgl. J. Chem. Soc. Perkin Trans 1, 589-591 (1977)):

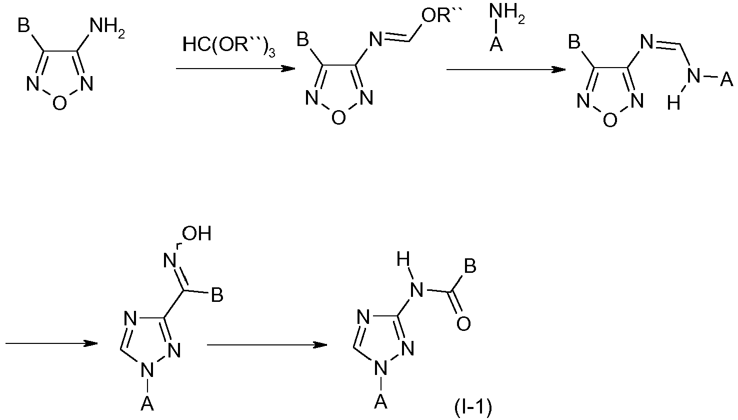

Die in dieser Synthesesequenz als Edukte verwendeten Amino-oxdiazole sind kommerziell erhältlich oder lassen sich nach literaturbekannten Methoden herstellen. Siehe zum Beispiel WO 2009/0011850, Heterocycles 57, 811 (2002), WO 2011/132017, J. Med. Chem. 23, 690-692 (1980), Heterocycles 34, 2313-2322 (1992), Tetrahedron 57, 5865-5871 (2001), J. Chem. Soc. Perkin Trans 1, 1313 (1988), Z. Chem. 14, 94 (1974).

Beispielhaft ist die Synthese erfindungsgemäßer Verbindungen (der besseren Übersichtlichkeit wegen werden mögliche Reste R2, R3 und R7 nicht dargestellt) anhand der Herstellung der Verbindungen der Formel (Ia-1) im Syntheseschema 1 dargestellt (vgl. hierzu auch die Herstellbeispiele).

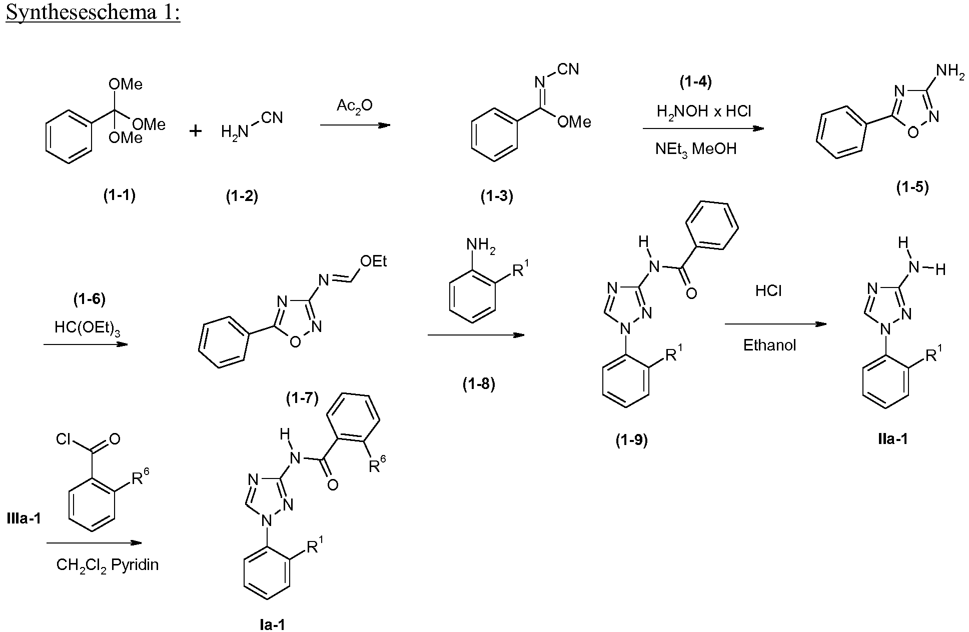

Die auf diesem Wege synthetisierten 3-Benzamido-1-aryl-1,2,4-triazole (1-9) sind neu und auch Gegenstand der Erfindung.

Ausgehend von substituierten 3-Amino-5-aryl-1,2,4-oxadiazolen der Formel (2-6), die sich mit Hilfe literaturbekannter Methoden herstellen lassen (vgl. Heterocycles, 57, 811-823), lassen sich die Verbindungen der Formel (Ia-1) durch Umsetzung entsprechend substituierter Alkoxy-[5-aryl-1,2,4-oxadiazol-3-yl-]imidoformiaten der Formel (2-8) mit Anilinen der Formel (2-9) gemäß Syntheseschema 2 herstellen.

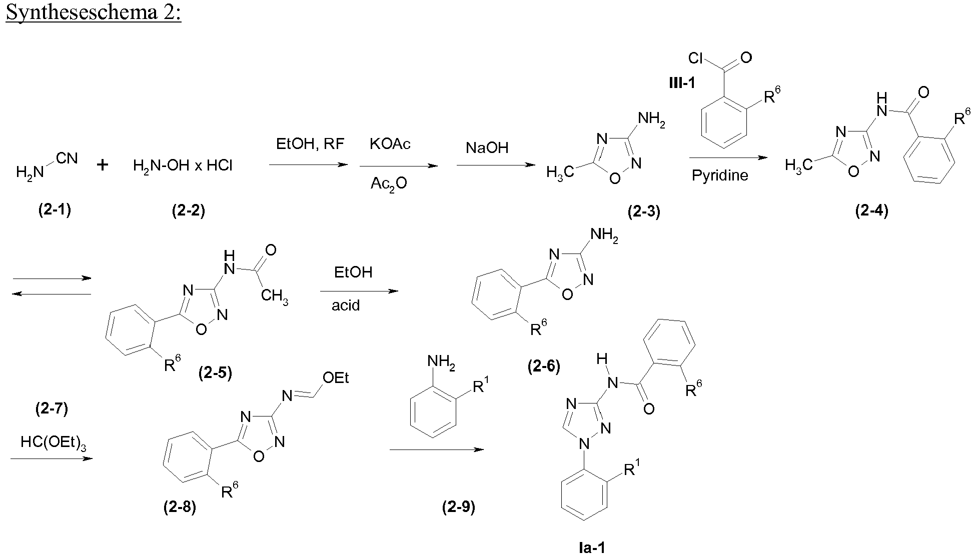

Verbindungen der Formeln (I-2) 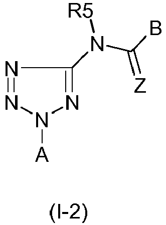 können auch gemäß Syntheseschema 3 erhalten werden:

### Syntheseschema 3:

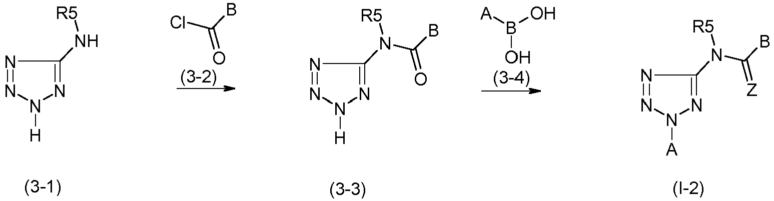

Aminotetrazole der Formel (3-1) können mit einem Carbonsäurechlorid der Formel (3-2) zu einem Amid mit der Struktur (3-3) umgesetzt werden. Die Reaktion von 2-H-Tetrazolen der Formel (3-3) mit Boronsäuren der Formel (3-4) in Gegenwart von Kupfer(II)verbindungen (z.B. Kupfer(II)acetat und Pyridin, vgl. Tetrahedron 1998, 39, 2941) liefert Verbindungen der Formel (I-2).

Amine der Formel (II-2) 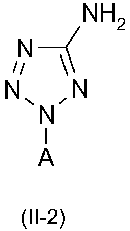 sind zum Teil kommerziell erhältlich. Als Beispiel sei 2-Phenyl-2H-tetrazol-5-amin genannt.

Neue Amine der Formel (II-2) können gemäß Syntheseschema 4 erhalten werden:

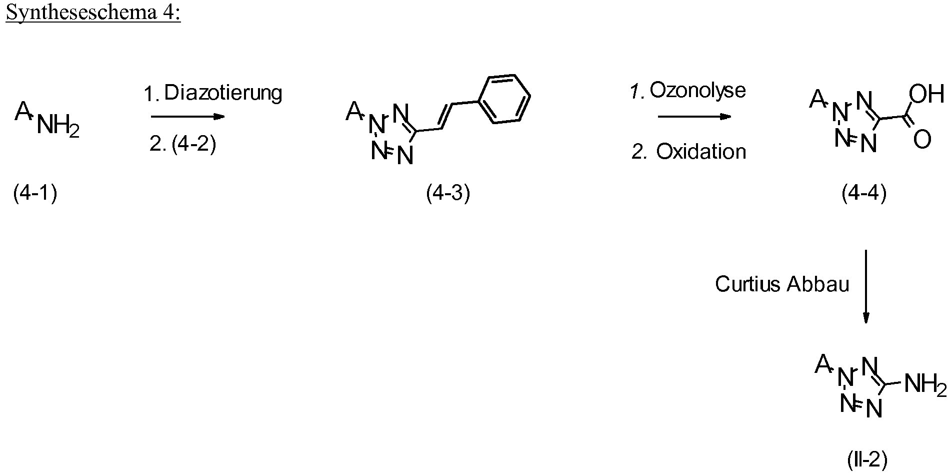

Aromatische Aniline der Formel (4-1) können in erstem Schritt diazotiert werden, z.B. mit Natriumnitrit in Salzsäure, und anschließend mit einem Tosylhydrazon, wie z.B. (4-2) der Formel 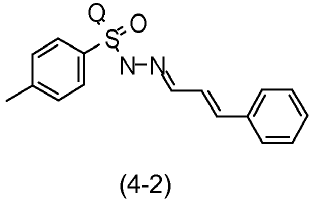 in Gegenwart von Pyridin zum Tetrazol der Formel (4-3) zyklisiert werden (vgl. J. Med. Chem. 2000, 43, 953). Die Verbindung der Formel (4-3) liefert nach Ozonolyse und nachfolgender Oxidation (z.B. mit Natriumchromat(VI), vgl. Comprehensive Organic Transformations: A Guide to Functional Group Preparations; Larock, R. C., Ed.; Wiley-VCH: New York, 1999) die Carbonsäure der Formel (4-4), die durch Curtius-Abbau in das 5-Amino-tetrazol der Formel (II-2) überführt werden kann (vgl. Org. React. 1946, 337).

Die erfindungsgemäßen Verfahren zur Herstellung der neuen Verbindungen der Formel (I) werden vorzugsweise unter Verwendung eines Verdünnungsmittels durchgeführt. Als Verdünnungsmittel zur Durchführung der erfindungsgemäßen Verfahren kommen neben Wasser alle inerten Lösungsmittel in Frage. Als Beispiele sind zu nennen: Halogenkohlenwasserstoffe (z.B. Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, 1,4-Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, *o-*Nitrotoluol); Nitrile (wie z.B. Acetonitril, Propionitril, Butyronitril, Isobutyronitril, Benzonitril, *m-*Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Octan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeintervalles von 70°C bis 190°C, Cyclohexan, Methylcyclohexan, Petrolether, Ligroin, Benzol, Toluol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäure-triamid, Formamid, N-Methylformamid, N,N-Dimethylformamid, N,N-Dipropylformamid, N,N-Dibutylformamid, N-Methylpyrrolidin, N-Methylcaprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formylpiperidin, N,N'-Diformylpiperazin) und Ketone (z.B. Aceton, Acetophenon, Methylethylketon, Methylbutylketon).

Selbstverständlich kann man das erfindungsgemäße Verfahren auch in Gemischen der genannten Lösungs- und Verdünnungsmittel durchführen.

Die Reaktionstemperaturen können bei der Durchführung des erfindungsgemäßen Verfahrens in einem größeren Bereich variiert werden. Im Allgemeinen arbeitet man bei Temperaturen zwischen -30 °C und +150 °C, vorzugsweise zwischen -10 °C und +100 °C.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck - im Allgemeinen bei absoluten Drücken zwischen 0,1 bar und 15 bar - durchzuführen.

Zur Durchführung des erfindungsgemäßen Verfahrens werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels, gegebenenfalls auch unter einer Schutzgasatmosphäre (z.B. unter Stickstoff, Argon oder Helium) durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Natriums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)-octan (DABCO), 1,8-Diazabicyclo(5.4.0)undecen (DBU), Cyclohexyltetrabutyl-guanidin (CyTBG), Cyclohexyltetramethylguanidin (CyTMG), N,N,N,N-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, N,N-Dimethylanilin, N,N-Dimethyltoluidin, N,N-Dimethyl-*p*-aminopyridin, N-Methyl-pyrrolidin, N-Methylpiperidin, N-Methyl-imidazol, N-Methyl-pyrazol, N-Methyl-morpholin, N-Methyl-hexamethylendiamin, Pyridin, 4-Pyrrolidinopyridin, 4-Dimethylamino-pyridin, Chinolin, 2-Picolin, 3-Picolin, Pyrimidin, Acridin, N,N,N',N'-Tetramethylendiamin, N,N,N',N'-Tetraethylendiamin, Chinoxalin, N-Propyldiisopropylamin, N-Ethyl-diisopropylamin, N,N'-Dimethyl-cyclohexylamin, 2,6-Lutidin, 2,4-Lutidin oder Triethyldiamin).

Als saure Reaktionshilfsmittel zur Durchführung der erfindungsgemäßen Verfahren können alle Mineralsäuren (z.B. Halogenwasserstoffsäuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)chlorid, Bortrifluorid oder sein Etherat, Titan(IV)chlorid, Zinn(IV)chlorid) und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure) eingesetzt werden.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereo¬isomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Linepithema humile, Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

### Nematoden

Der Begriff "Nematoden" umfasst im vorliegenden Zusammenhang alle Arten des Stammes Nematoda und hierbei insbesondere Arten, die Pflanzen oder Pilze (zum Beispiel Arten der Ordnung Aphelenchida, Meloidogyne, Tylenchida und andere) oder auch Menschen und Tiere (zum Beispiel Arten der Ordnungen Trichinellida, Tylenchida, Rhabditida und Spirurida) parasitieren oder in bzw. an diesen Lebewesen Schädigungen verursachen, sowie andere parasitäre Helminthen.

Ein Nematizid im Pflanzenschutz, wie hier beschrieben, besitzt die Fähigkeit, Nematoden zu kontrollieren.

Der Begriff "Nematoden kontrollieren" bedeutet das Abtöten der Nematoden oder das Verhindern oder Erschweren ihrer Entwicklung bzw. ihres Wachstums oder das Verhindern oder Erschweren ihres Eindringens in oder ihres Saugens am pflanzlichen Gewebe.

Dabei wird die Wirksamkeit der Verbindungen durch einen Vergleich von Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden zwischen einer mit der Verbindung der Formel (I) behandelten Pflanze, Pflanzenteil oder dem behandelten Boden und einer unbehandelten Pflanze, Pflanzenteil oder unbehandelten Boden (100 %) ermittelt. Vorzugsweise wird eine Verringerung um 25-50 % im Vergleich mit einer unbehandelten Pflanze, Pflanzenteil oder unbehandelten Boden, besonders bevorzugt eine Verringerung um 51 - 79 % und ganz besonders bevorzugt das vollständige Abtöten oder die vollständige Verhinderung von Entwicklung und Wachstum der Nematoden durch eine Verringerung um 80 bis 100 % erreicht. Die Kontrolle von Nematoden, wie hier beschreiben, beinhaltet ebenso die Kontrolle der Nematoden-Vermehrung (Entwicklung von Zysten und/oder Eier). Verbindungen der Formel (I) können ebenso verwendet werden, um die Pflanzen oder Tiere gesund zu erhalten und können kurativ, präventiv oder systemisch zur Nematoden-Kontrolle eingesetzt werden.

Dem Fachmann sind Methoden bekannt, wie Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden bestimmt werden.

Die Verwendung einer Verbindung der Formel (I) kann die Pflanze gesund erhalten und beinhaltet ebenso eine Reduktion der von Nematoden hervorgerufenen Schäden sowie eine Erhöhung der Erntemenge.

Der Begriff "Nematoden" bezieht sich im vorliegenden Zusammenhang auf Pflanzennematoden, unter die man alle Nematoden zusammenfasst, die Pflanzen schädigen. Pflanzennematoden umfassen pflanzenparasitäre Nematoden und im Boden lebende Nematoden. Zu den pflanzenparasitären Nematoden zählen Ektoparasiten wie Xiphinema spp., Longidorus spp. und Trichodorus spp.; Halbparasiten wie Tylenchulus spp.; migratorische Endoparasiten wie Pratylenchus spp., Radopholus spp. und Scutellonema spp.; ortsgebundene Parasiten wie Heterodera spp., Globodera spp. und Meloidogyne spp., sowie Stängel- und Blattendoparasiten wie Ditylenchus spp., Aphelenchoides spp. und Hirschmaniella spp.. Besonders schädliche wurzelparasitäre Bodennematoden sind zum Beispiel zystenbildende Nematoden der Gattungen Heterodera oder Globodera, und/oder Wurzelgallennematoden der Gattung Meloidogyne. Schädliche Arten dieser Gattungen sind zum Beispiel Meloidogyne incognita, Heterodera glycines (Sojabohnenzystennematode), Globodera pallida und Globodera rostochiensis (Kartoffelzystennematode), wobei diese Arten wirksam mit dem im vorliegenden Text beschriebenen Verbindungen bekämpft werden. Die Verwendung der im vorliegenden Text beschriebenen Verbindungen ist jedoch keineswegs auf diese Gattungen oder Arten beschränkt, sondern erstreckt sich in gleicher Weise auch auf andere Nematoden.

Zu den Pflanzennematoden zählen z.B. Aglenchus agricola, Anguina tritici, Aphelenchoides arachidis, Aphelenchoides fragaria und die Stängel- und Blattendoparasiten Aphelenchoides spp., Belonolaimus gracilis, Belonolaimus longicaudatus, Belonolaimus nortoni, Bursaphelenchus cocophilus, Bursaphelenchus eremus, Bursaphelenchus xylophilus und Bursaphelenchus spp., Cacopaurus pestis, Criconemella curvata, Criconemella onoensis, Criconemella ornata, Criconemella rusium, Criconemella xenoplax (= Mesocriconema xenoplax) und Criconemella spp.,

Criconemoides ferniae, Criconemoides onoense, Criconemoides ornatum und Criconemoides spp., Ditylenchus destructor, Ditylenchus dipsaci, Ditylenchus myceliophagus sowie die Stängel- und Blattendoparasiten Ditylenchus spp., Dolichodorus heterocephalus, Globodera pallida (=Heterodera pallida), Globodera rostochiensis (Kartoffelzystennematode), Globodera solanacearum, Globodera tabacum, Globodera virginia und die ortsgebundenen zystenbildenden Parasiten Globodera spp., Helicotylenchus digonicus, Helicotylenchus dihystera, Helicotylenchus erythrine, Helicotylenchus multicinctus, Helicotylenchus nannus, Helicotylenchus pseudorobustus und Helicotylenchus spp., Hemicriconemoides, Hemicycliophora arenaria, Hemicycliophora nudata, Hemicycliophora parvana, Heterodera avenae, Heterodera cruciferae, Heterodera glycines (Sojabohnenzystennematode), Heterodera oryzae, Heterodera schachtii, Heterodera zeae und die ortsgebundenen zystenbildenden Parasiten Heterodera spp., Hirschmaniella gracilis, Hirschmaniella oryzae, Hirschmaniella spinicaudata und die Stängel- und Blattendoparasiten Hirschmaniella spp., Hoplolaimus aegyptii, Hoplolaimus californicus, Hoplolaimus columbus, Hoplolaimus galeatus, Hoplolaimus indicus, Hoplolaimus magnistylus, Hoplolaimus pararobustus, Longidorus africanus, Longidorus breviannulatus, Longidorus elongatus, Longidorus laevicapitatus, Longidorus vineacola und die Ektoparasiten Longidorus spp., Meloidogyne acronea, Meloidogyne africana, Meloidogyne arenaria, Meloidogyne arenaria thamesi, Meloidogyne artiella, Meloidogyne chitwoodi, Meloidogyne coffeicola, Meloidogyne ethiopica, Meloidogyne exigua, Meloidogyne fallax, Meloidogyne graminicola, Meloidogyne graminis, Meloidogyne hapla, Meloidogyne incognita, Meloidogyne incognita acrita, Meloidogyne javanica, Meloidogyne kikuyensis, Meloidogyne minor, Meloidogyne naasi, Meloidogyne paranaensis, Meloidogyne thamesi und die ortsgebundenen Parasiten Meloidogyne spp., Meloinema spp., Nacobbus aberrans, Neotylenchus vigissi, Paraphelenchus pseudoparietinus, Paratrichodorus allius, Paratrichodorus lobatus, Paratrichodorus minor, Paratrichodorus nanus, Paratrichodorus porosus, Paratrichodorus teres und Paratrichodorus spp., Paratylenchus hamatus, Paratylenchus minutus, Paratylenchus projectus und Paratylenchus spp., Pratylenchus agilis, Pratylenchus alleni, Pratylenchus andinus, Pratylenchus brachyurus, Pratylenchus cerealis, Pratylenchus coffeae, Pratylenchus crenatus, Pratylenchus delattrei, Pratylenchus giibbicaudatus, Pratylenchus goodeyi, Pratylenchus hamatus, Pratylenchus hexincisus, Pratylenchus loosi, Pratylenchus neglectus, Pratylenchus penetrans,Pratylenchus pratensis, Pratylenchus scribneri, Pratylenchus teres, Pratylenchus thornei, Pratylenchus vulnus, Pratylenchus zeae und die migratorischen Endoparasiten Pratylenchus spp., Pseudohalenchus minutus, Psilenchus magnidens, Psilenchus tumidus, Punctodera chalcoensis, Quinisulcius acutus, Radopholus citrophilus, Radopholus similis, die migratorischen Endoparasiten Radopholus spp., Rotylenchulus borealis, Rotylenchulus parvus, Rotylenchulus reniformis und Rotylenchulus spp., Rotylenchus laurentinus, Rotylenchus macrodoratus, Rotylenchus robustus, Rotylenchus uniformis und Rotylenchus spp., Scutellonema brachyurum, Scutellonema bradys, Scutellonema clathricaudatum und die migratorischen Endoparasiten Scutellonema spp., Subanguina radiciola, Tetylenchus nicotianae, Trichodorus cylindricus, Trichodorus minor, Trichodorus primitivus, Trichodorus proximus, Trichodorus similis, Trichodorus sparsus und die Ektoparasiten Trichodorus spp., Tylenchorhynchus agri, Tylenchorhynchus brassicae, Tylenchorhynchus clarus, Tylenchorhynchus claytoni, Tylenchorhynchus digitatus, Tylenchorhynchus ebriensis, Tylenchorhynchus maximus, Tylenchorhynchus nudus, Tylenchorhynchus vulgaris und Tylenchorhynchus spp., Tylenchulus semipenetrans und die Semiparasiten Tylenchulus spp., Xiphinema americanum, Xiphinema brevicolle, Xiphinema dimorphicaudatum, Xiphinema index und die Ektoparasiten Xiphinema spp.

Zu den Nematoden, zu deren Bekämpfung eine Verbindung der Formel (I) eingesetzt werden kann, zählen Nematoden der Gattung Meloidogyne wie der Southern Root-Knot Nematode (Meloidogyne incognita), der Javanese Root-Knot Nematode (Meloidogyne javanica, der Northern Root-Knot Nematode (Meloidogyne hapla) und der Peanut Root-Knot Nematode (Meloidogyne arenaria); Nematoden der Gattung Ditylenchus wie das Kartoffelkrätzeälchen (Ditylenchus destructor) und das Stock- und Stängelälchen (Ditylenchus dipsaci); Nematoden der Gattung Pratylenchus wie der Cob Root-Lesion Nematode (Pratylenchus penetrans), der Chrysanthemum Root-Lesion Nematode (Pratylenchus fallax), der Kaffeewurzelnematode (Pratylenchus coffeae), der Teewurzelnematode (Pratylenchus loosi) und der Walnut Root-Lesion Nematode (Pratylenchus vulnus); Nematoden der Gattung Globodera wie das Goldfarbene Kartoffelzystenälchen (Globodera rostochiensis) und das Weiße Kartoffelzystenälchen (Globodera pallida); Nematoden der Gattung Heterodera wie der Sojabohnenzystennematode (Heterodera glycines) und das Rübenzystenälchen (Heterodera schachtii); Nematoden der Gattung Aphelenchoides wie der Rice White-tip Nematode (Aphelenchoides besseyi), das Chrysanthemenälchen (Aphelenchoides ritzemabosi) und das Erdbeerälchen (Aphelenchoides fragariae); Nematoden der Gattung Aphelenchus wie der fungivore Nematode (Aphelenchus avenae); Nematoden der Gattung Radopholus, wie der Burrowing-Nematode (Radopholus similis); Nematoden der Gattung Tylenchulus wie der Orangenwurzelnematode (Tylenchulus semipenetrans); Nematoden der Gattung Rotylenchulus wie der reniforme Nematode (Rotylenchulus reniformis); in Bäumen lebende Nematoden, wie der Kiefernholznematode (Bursaphelenchus xylophilus) und der Red Ring Nematode (Bursaphelenchus cocophilus) und dergleichen.

Zu den Pflanzen, zu deren Schutz eine Verbindung der Formel (I) verwendet werden kann, zählen Pflanzen wie Getreide (zum Beispiel Reis, Gerste, Weizen, Roggen, Hafer, Mais, und dergleichen), Bohnen (Sojabohne, Azukibohne, Bohne, Dicke Bohne, Erbsen, Erdnüsse und dergleichen), Obstbäume/Früchte (Äpfel, Zitrusarten, Birnen, Trauben, Pfirsiche, japanische Aprikosen, Kirschen, Walnüsse, Mandeln, Bananen, Erdbeeren und dergleichen), Gemüsearten (Kohl, Tomate, Spinat, Brokkoli, Salat, Zwiebel, Röhrenlauch, Pfeffer und dergleichen), Hackfrüchte (Karotte, Kartoffel, Süßkartoffel, Rettich, Lotuswurzel, Steckrübe und dergleichen), Pflanzen für industrielle Rohstoffe (Baumwolle, Hanf, Papiermaulbeere, Mitsumata, Raps, Rübe, Hopfen, Zuckerrohr, Zuckerrübe, Olive, Gummi, Palmen, Kaffee, Tabak, Tee und dergleichen), Kürbisgewächse (Kürbis, Gurke, Wassermelone, Melone und dergleichen), Weidepflanzen (Knaulgras, Sorgum, Wiesenlieschgras, Klee, Luzerne und dergleichen), Rasengräser (Maskarenengras, Straußgras und dergleichen), Gewürzpflanzen usw. (Lavendel, Rosmarin, Thymian, Petersilie, Pfeffer, Ingwer und dergleichen) und Blumenpflanzen (Chrysantheme, Rose, Orchidee und dergleichen).

Die Verbindungen der Formel (I) eignen sich besonders für die Bekämpfung von Nematoden des Kaffees, insbesondere von Pratylenchus brachyurus, Pratylenchus coffeae, Meloidogyne exigua, Meloidogyne incognita, Meloidogyne coffeicola, Helicotylenchus spp. sowie aus Meloidogyne paranaensis, Rotylenchus spp., Xiphinema spp., Tylenchorhynchus spp. und Scutellonema spp..

Die Verbindungen der Formel (I) eignen sich besonders für die Bekämpfung von Nematoden der Kartoffel, insbesondere von Pratylenchus brachyurus, Pratylenchus pratensis, Pratylenchus scribneri, Pratylenchus penetrans, Pratylenchus coffeae, Ditylenchus dipsaci sowie aus Pratylenchus alleni, Pratylenchus andinus, Pratylenchus cerealis, Pratylenchus crenatus, Pratylenchus hexincisus, Pratylenchus loosi, Pratylenchus neglectus, Pratylenchus teres, Pratylenchus thornei, Pratylenchus vulnus, Belonolaimus longicaudatus, Trichodorus cylindricus, Trichodorus primitivus, Trichodorus proximus, Trichodorus similis, Trichodorus sparsus, Paratrichodorus minor, Paratrichodorus allius, Paratrichodorus nanus, Paratrichodorus teres, Meloidogyne arenaria, Meloidogyne fallax, Meloidogyne hapla, Meloidogyne thamesi, Meloidogyne incognita, Meloidogyne chitwoodi, Meloidogyne javanica, Nacobbus aberrans, Globodera rostochiensis, Globodera pallida, Ditylenchus destructor, Radopholus similis, Rotylenchulus reniformis, Neotylenchus vigissi, Paraphelenchus pseudoparietinus, Aphelenchoides fragariae und Meloinema spp.

Die Verbindungen der Formel (I) eignen sich besonders für die Bekämpfung von Nematoden der Tomate, insbesondere von Meloidogyne arenaria, Meloidogyne hapla, Meloidogyne javanica, Meloidogyne incognita, Pratylenchus penetrans und auch Pratylenchus brachyurus, Pratylenchus coffeae, Pratylenchus scribneri, Pratylenchus vulnus, Paratrichodorus minor, Meloidogyne exigua, Nacobbus aberrans, Globodera solanacearum, Dolichodorus heterocephalus und Rotylenchulus reniformis.

Die Verbindungen der Formel (I) eignen sich besonders für die Bekämpfung von Nematoden von Gurkengewächsen, insbesondere von Meloidogyne arenaria, Meloidogyne hapla, Meloidogyne javanica, Meloidogyne incognita, Rotylenchulus reniformis und Pratylenchus thornei.

Die Verbindungen der Formel (I) eignen sich besonders für die Bekämpfung von Nematoden der Baumwolle, insbesondere von Belonolaimus longicaudatus, Meloidogyne incognita, Hoplolaimus columbus, Hoplolaimus galeatus und Rotylenchulus reniformis.

Die Verbindungen der Formel (I) eignen sich besonders für die Bekämpfung von Nematoden des Maises, insbesondere von Belonolaimus longicaudatus, Paratrichodorus minor und auch Pratylenchus brachyurus, Pratylenchus delattrei, Pratylenchus hexincisus, Pratylenchus penetrans, Pratylenchus zeae, (Belonolaimus gracilis), Belonolaimus nortoni, Longidorus breviannulatus, Meloidogyne arenaria, Meloidogyne arenaria thamesi, Meloidogyne graminis, Meloidogyne incognita, Meloidogyne incognita acrita, Meloidogyne javanica, Meloidogyne naasi, Heterodera avenae, Heterodera oryzae, Heterodera zeae, Punctodera chalcoensis, Ditylenchus dipsaci, Hoplolaimus aegyptii, Hoplolaimus magnistylus, Hoplolaimus galeatus, Hoplolaimus indicus, Helicotylenchus digonicus, Helicotylenchus dihystera, Helicotylenchus pseudorobustus, Xiphinema americanum, Dolichodorus heterocephalus, Criconemella ornata, Criconemella onoensis, Radopholus similis, Rotylenchulus borealis, Rotylenchulus parvus, Tylenchorhynchus agri, Tylenchorhynchus clarus, Tylenchorhynchus claytoni, Tylenchorhynchus maximus, Tylenchorhynchus nudus, Tylenchorhynchus vulgaris, Quinisulcius acutus, Paratylenchus minutus, Hemicycliophora parvana, Aglenchus agricola, Anguina tritici, Aphelenchoides arachidis, Scutellonema brachyurum und Subanguina radiciola.

Die Verbindungen der Formel (I) eignen sich besonders für die Bekämpfung von Nematoden der Sojabohne, insbesondere vonPratylenchus brachyurus, Pratylenchus pratensis, Pratylenchus penetrans, Pratylenchus scribneri, Belonolaimus longicaudatus, Heterodera glycines, Hoplolaimus columbus und auch Pratylenchus coffeae, Pratylenchus hexincisus, Pratylenchus neglectus, Pratylenchus crenatus, Pratylenchus alleni, Pratylenchus agilis, Pratylenchus zeae, Pratylenchus vulnus, (Belonolaimus gracilis), Meloidogyne arenaria, Meloidogyne incognita, Meloidogyne javanica, Meloidogyne hapla, Hoplolaimus columbus, Hoplolaimus galeatus und Rotylenchulus reniformis.

Die Verbindungen der Formel (I) eignen sich besonders für die Bekämpfung von Nematoden des Tabaks, insbesondere von Meloidogyne incognita, Meloidogyne javanica und auch Pratylenchus brachyurus, Pratylenchus pratensis, Pratylenchus hexincisus, Pratylenchus penetrans, Pratylenchus neglectus, Pratylenchus crenatus, Pratylenchus thornei, Pratylenchus vulnus, Pratylenchus zeae, Longidorus elongatu, Paratrichodorus lobatus, Trichodorus spp., Meloidogyne arenaria, Meloidogyne hapla, Globodera tabacum, Globodera solanacearum, Globodera virginiae, Ditylenchus dipsaci, Rotylenchus spp., Helicotylenchus spp., Xiphinema americanum, Criconemella spp., Rotylenchulus reniformis, Tylenchorhynchus claytoni, Paratylenchus spp. und Tetylenchus nicotianae.

Die Verbindungen der Formel (I) eignen sich besonders für die Bekämpfung von Nematoden von Zitrusgewächsen, insbesondere von Pratylenchus coffeae und auch Pratylenchus brachyurus, Pratylenchus vulnus, Belonolaimus longicaudatus, Paratrichodorus minor, Paratrichodorus porosus, Trichodorus , Meloidogyne incognita, Meloidogyne incognita acrita, Meloidogyne javanica, Rotylenchus macrodoratus, Xiphinema americanum, Xiphinema brevicolle, Xiphinema index, Criconemella spp., Hemicriconemoides, Radopholus similis bzw. Radopholus citrophilus, Hemicycliophora arenaria, Hemicycliophora nudata und Tylenchulus semipenetrans .

Die Verbindungen der Formel (I) eignen sich besonders für die Bekämpfung von Nematoden der Banane, insbesondere von Pratylenchus coffeae, Radopholus similis und auch Pratylenchus giibbicaudatus, Pratylenchus loosi, Meloidogyne spp., Helicotylenchus multicinctus, Helicotylenchus dihystera und Rotylenchulus spp..

Die Verbindungen der Formel (I) eignen sich besonders für die Bekämpfung von Nematoden der Ananas, insbesondere von Pratylenchus zeae, Pratylenchus pratensis, Pratylenchus brachyurus, Pratylenchus goodeyi., Meloidogyne spp., Rotylenchulus reniformis und auch Longidorus elongatus, Longidorus laevicapitatus, Trichodorus primitivus, Trichodorus minor, Heterodera spp., Ditylenchus myceliophagus, Hoplolaimus californicus, Hoplolaimus pararobustus, Hoplolaimus indicus, Helicotylenchus dihystera, Helicotylenchus nannus, Helicotylenchus multicinctus, Helicotylenchus erythrine, Xiphinema dimorphicaudatum, Radopholus similis, Tylenchorhynchus digitatus, Tylenchorhynchus ebriensis, Paratylenchus minutus, Scutellonema clathricaudatum, Scutellonema bradys, Psilenchus tumidus, Psilenchus magnidens, Pseudohalenchus minutus, Criconemoides ferniae, Criconemoides onoense und Criconemoides ornatum.

Die Verbindungen der Formel (I) eignen sich besonders für die Bekämpfung von Nematoden von Trauben, insbesondere von Pratylenchus vulnus, Meloidogyne arenaria, Meloidogyne incognita, Meloidogyne javanica, Xiphinema americanum, Xiphinema index und auch Pratylenchus pratensis, Pratylenchus scribneri, Pratylenchus neglectus, Pratylenchus brachyurus, Pratylenchus thornei und Tylenchulus semipenetrans.

Die Verbindungen der Formel (I) eignen sich besonders für die Bekämpfung von Nematoden von Baumkulturen - Kernobst, insbesondere von Pratylenchus penetrans und auch Pratylenchus vulnus, Longidorus elongatus, Meloidogyne incognita und Meloidogyne hapla.

Die Verbindungen der Formel (I) eignen sich besonders für die Bekämpfung von Nematoden von Baumkulturen - Steinfrüchten, insbesondere von Pratylenchus penetrans, Pratylenchus vulnus, Meloidogyne arenaria, Meloidogyne hapla, Meloidogyne javanica, Meloidogyne incognita, Criconemella xenoplax und auch Pratylenchus brachyurus, Pratylenchus coffeae, Pratylenchus scribneri, Pratylenchus zeae, Belonolaimus longicaudatus, Helicotylenchus dihystera, Xiphinema americanum, Criconemella curvata, Tylenchorhynchus claytoni, Paratylenchus hamatus, Paratylenchus projectus, Scutellonema brachyurum und Hoplolaimus galeatus.

Die Verbindungen der Formel (I) eignen sich besonders für die Bekämpfung von Nematoden in Baumkulturen, Zuckerrohr und Reis, insbesondere von Trichodorus spp., Criconemella spp. und auch Pratylenchus spp. , Paratrichodorus spp., Meloidogyne spp. , Helicotylenchus spp., Tylenchorhynchus spp., Aphelenchoides spp. Heterodera spp, Xiphinema spp. und Cacopaurus pestis.

Ebenso bezieht sich der Begriff "Nematoden" im vorliegenden Zusammenhang auf Nematoden, die Menschen oder Tiere schädigen.

Spezifische Nematodenarten, die für den Menschen oder für Tiere schädlich sind, sind:
Trichinellida, zum Beispiel: Trichuris spp., Capillaria spp., Paracapillaria spp., Eucoleus spp., Trichomosoides spp., Trichinella spp.

Aus der Ordnung der Tylenchida zum Beispiel: Micronema spp., Strongyloides spp.

Aus der Ordnung der Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.

Aus der Odnung der Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;

Viele bekannte Nematizide wirken gleichsam gegen andere parasitäre Helminthen und werden daher für die Bekämpfung von human- und tierparasitären Würmern, die nicht unbedingt zu der Gruppe Nematoda gehören, verwendet. Die vorliegende Erfindung bezieht sich auch auf die Verwendung der Verbindungen der Formel (I) als anthelmintische Arzneimittel. Zu den pathogenen endoparasitären Helminthen zählen Platyhelmintha (z.B. Monogenea, Cestodes und Trematodes), Acanthocephala und Pentastoma. Die folgenden Helminthen sind als bevorzugt zu erwähnen:
Monogenea: z.B.: Gyrodactylus spp., Dactylogyrus spp., Polystoma spp.
Cestodes: aus der Ordnung der Pseudophyllidea zum Beispiel: Diphyllobothrium spp., Spirometra spp., Schistocephalus spp., Ligula spp., Bothridium spp., Diplogonoporus spp.

Aus der Ordnung der Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Trematodes: aus der Klasse der Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonimus spp.

Acanthocephala: aus der Ordnung der Oligacanthorhynchida z.B.: Macracanthorhynchus spp., Prosthenorchis spp.; aus der Ordnung der Polymorphida zum Beispiel: Filicollis spp.; aus der Ordnung der Moniliformida zum Beispiel: Moniliformis spp.,

Aus der Ordnung der Echinorhynchida zum Beispiel Acanthocephalus spp., Echinorhynchus spp., Leptorhynchoides spp.

Pentastoma: aus der Ordnung der Porocephalida zum Beispiel Linguatula spp.

Auf dem Gebiet der Tiermedizin und in der Tierhaltung erfolgt die Verabreichung der Verbindungen der Formel (I) auf bekannte Weise direkt oder enteral, parenteral, dermal oder nasal in Form von geeigneten Anwendungsformen. Die Verabreichung kann prophylaktisch oder therapeutisch erfolgen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide,
   weitere Wirkstoffe wie beispielsweise Afidopyropen, Azadirachtin, Benclothiaz, Benzoximate, Bifenazate, Bromopropylate, Chinomethionat, Cryolite,
   Dicofol, Diflovidazin, Fluensulfone, Flometoquin, Flufenerim, Flufenoxystrobin, Flufiprole, Fluopyram, Flupyradifurone, Fufenozide, Heptafluthrin, Imidaclothiz, Iprodione, Meperfluthrin, Paichongding, Pyflubumide, Pyrifluquinazon, Pyriminostrobin, Tetramethylfluthrin und Iodmethan; desweiteren Präparate auf Basis von Bacillus firmus (I-1582, BioNeem, Votivo), sowie folgende Verbindungen:3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) und 1-{2-Fluor-4-methyl-5-[(2,2,2-trifluorethyl)sulfinyl]phenyl} -3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{5-[3-Chlor-5-(trifluormethyl)phenyl]-5-(trifluormethyl)-4,5-dihydro-1,2-oxazol-3-yl}-N-{2-oxo-2-[(2,2,2-trifluorethyl)amino]ethyl}-1-naphthamid (bekannt aus WO2009/002809), Methyl-2-[2-({[3-brom-1-(3-chlorpyridin-2-yl)-1H-pyrazol-5-yl]carbonyl}amino)-5-chlor-3-methylbenzoyl]-2-methylhydrazin-carboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl} amino)benzoyl]-2-ethyl-hydrazincarboxylat (bekannt aus WO2005/085216), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 3-Chlor-N-(2-cyanpropan-2-yl)-N-[4-(1,1,1,2,3,3,3-heptafluorpropan-2-yl)-2-methylphenyl]phthalamid (bekannt aus WO2012/034472), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), (5S,8R)-1-[(6-Chlorpyridin-3-yl)methyl]-9-nitro-2,3,5,6,7,8-hexahydro-1H-5,8-epoxyimidazo[1,2-a]azepin (bekannt aus WO2010/069266), (2E)-1-[(6-Chlorpyridin-3-yl)methyl]-N'-nitro-2-pentylidenhydrazincarboximidamid (bekannt aus WO2010/060231), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).
(1) Inhibitoren der Ergosterol-Biosynthese, wie beispielsweise (1.1) Aldimorph, (1.2) Azaconazol, (1.3) Bitertanol, (1.4) Bromuconazol, (1.5) Cyproconazol, (1.6) Diclobutrazol, (1.7) Difenoconazol, (1.8) Diniconazol, (1.9) Diniconazol-M, (1.10) Dodemorph, (1.11) Dodemorph Acetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-Cis, (1.25) Hexaconazol, (1.26) Imazalil, (1.27) Imazalil Sulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) Methyl-1-(2,2-dimethyl-2,3-dihydro-1H-inden-1-yl)-1H-imidazol-5-carboxylat, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imido-formamid und (1.64) O-[1-(4-Methoxyphenoxy)-3,3-dimethylbutan-2-yl]-1H-imidazol-1-carbothioat, (1.65) Pyrisoxazole.
(2) Inhibitoren der Respiration (Atmungsketten-Inhibitoren), wie beispielsweise (2.1) Bixafen, (2.2) Boscalid, (2.3) Carboxin, (2.4) Diflumetorim, (2.5) Fenfuram, (2.6) Fluopyram, (2.7) Flutolanil, (2.8) Fluxapyroxad, (2.9) Furametpyr, (2.10) Furmecyclox, (2.11) Isopyrazam Mischung des syn-epimeren Razemates 1RS,4SR,9RS und des anti-empimeren Razemates 1RS,4SR,9SR, (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxane, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4- {[4-(trifluormethyl)pyridin-2-yl]oxy}phenyl)ethyl]quinazolin-4-amin, (2.29) Benzovindiflupyr, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridine-3-carboxamid, (2.43) Isofetamid.
(3) Inhibitoren der Respiration (Atmungsketten-Inhibitoren) am Komplex III der Atumungskette, wie beispielsweise (3.1) Ametoctradin, (3.2) Amisulbrom, (3.3) Azoxystrobin, (3.4) Cyazofamid, (3.5) Coumethoxystrobin, (3.6) Coumoxystrobin, (3.5) Dimoxystrobin, (3.8) Enestroburin, (3.9) Famoxadon, (3.10) Fenamidon, (3.11) Flufenoxystrobin, (3.12) Fluoxastrobin, (3.13) Kresoxim-Methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2- {[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid, (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid, (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat, (3.31) N-(3-Ethyl-3,5,5-trimethylcyclohexyl)-3-(formylamino)-2-hydroxybenzamid, (3.32) 2-{2-[(2,5-Dimethylphenoxy)methyl]phenyl}-2-methoxy-N-methylacetamid,
(4) Inhibitoren der Mitose und Zellteilung, wie beispielsweise (4.1) Benomyl, (4.2) Carbendazim, (4.3) Chlorfenazol, (4.4) Diethofencarb, (4.5) Ethaboxam, (4.6) Fluopicolid, (4.7) Fuberidazol, (4.8) Pencycuron, (4.9) Thiabendazol, (4.10) Thiophanat-Methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin und (4.14) 3-Chlor-5-(6-chlorpyridin-3-yl)-6-methyl-4-(2,4,6-trifluorphenyl)pyridazin.
(5) Verbindungen mit Multisite-Aktivität, wie beispielsweise (5.1) Bordeauxmischung, (5.2) Captafol, (5.3) Captan, (5.4) Chlorthalonil, (5.5) Kupferzubereitungen wie Kupferhydroxid, (5.6) Kupfernaphthenat, (5.7) Kupferoxid, (5.8) Kupferoxychlorid, (5.9) Kupfersulfat, (5.10) Dichlofluanid, (5.11) Dithianon, (5.12) Dodine, (5.13) Dodine freie Base, (5.14) Ferbam, (5.15) Fluorfolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mankupfer, (5.23) Mancozeb, (5.24) Maneb, (5.25) Metiram, (5.26) Zinkmetiram, (5.27) Kupfer-Oxin, (5.28) Propamidin, (5.29) Propineb, (5.30) Schwefel und Schwefelzubereitungen wie beispielsweise Calciumpolysulfid, (5.31) Thiram, (5.32) Tolylfluanid, (5.33) Zineb, (5.34) Ziram und (5,35) Anilazin.
(6) Resistenzinduktoren, wie beispielsweise (6.1) Acibenzolar-S-Methyl, (6.2) Isotianil, (6.3) Probenazol, (6.4) Tiadinil und (6.5) Laminarin.
(7) Inhibitoren der Aminosäure- und Protein-Biosynthese, wie beispielsweise (7.1), (7.2) Blasticidin-S, (7.3) Cyprodinil, (7.4) Kasugamycin, (7.5) Kasugamycin Hydrochlorid Hydrat, (7.6) Mepanipyrim, (7.7) Pyrimethanil, (7.8) 3-(5-Fluor-3,3,4,4-tetramethyl-3,4-dihydroisochinolin-1-yl)chinolin und (7.9) Oxytetracyclin und (7.10) Streptomycin.
(8) Inhibitoren der ATP Produktion, wie beispielsweise (8.1) Fentin Acetat, (8.2) Fentin Chlorid, (8.3) Fentin Hydroxid und (8.4) Silthiofam.
(9) Inhibitoren der Zellwandsynthese, wie beispielsweise (9.1) Benthiavalicarb, (9.2) Dimethomorph, (9.3) Flumorph, (9.4) Iprovalicarb, (9.5) Mandipropamid, (9.6) Polyoxins, (9.7) Polyoxorim, (9.8) Validamycin A, (9.9) Valifenalat und (9.10) Polyoxin B.
(10) Inhibitoren der Lipid- und Membran-Synthese, wie beispielsweise (10.1) Biphenyl, (10.2) Chlorneb, (10.3) Dicloran, (10.4) Edifenphos, (10.5) Etridiazol, (10.6) Iodocarb, (10.7) Iprobenfos, (10.8) Isoprothiolan, (10.9) Propamocarb, (10.10) Propamocarb Hydrochlorid, (10.11) Prothiocarb" (10.12) Pyrazophos, (10.13) Quintozen, (10.14) Tecnazene und (10.15) Tolclofos-Methyl.
(11) Inhibitoren der Melanin-Biosynthese, wie beispielsweise (11.1) Carpropamid, (11.2) Diclocymet, (11.3) Fenoxanil, (11.4) Fthalid, (11.5) Pyroquilon, (11.6) Tricyclazol, und (11.7) 2,2,2-Trifluorethyl {3-methyl-1-[(4-methylbenzoyl)amino]butan-2-yl}carbamat.
(12) Inhibitoren der Nukleinsäuresynthese, wie beispielsweise (12.1) Benalaxyl, (12.2) Benalaxyl-M (Kiralaxyl), (12.3) Bupirimat, (12.4) Clozylacon, (12.5) Dimethirimol, (12.6) Ethirimol, (12.7) Furalaxyl, (12.8) Hymexazol, (12.9) Metalaxyl, (12.10) Metalaxyl-M (Mefenoxam), (12.11) Ofurace, (12.12) Oxadixyl, (12.13) Oxolinsäure und (12.14) Octhilinon.
(13) Inhibitoren der Signaltransduktion, wie beispielsweise (13.1) Chlozolinat, (13.2) Fenpiclonil, (13.3) Fludioxonil, (13.4) Iprodion, (13.5) Procymidon, (13.6) Quinoxyfen, (13.7) Vinclozolin und (13.8) Proquinazid.
(14) Entkoppler, wie beispielsweise (14.1) Binapacryl, (14.2) Dinocap, (14.3) Ferimzon, (14.4) Fluazinam und (14.5) Meptyldinocap.
(15) Weitere Verbindungen, wie beispielsweise (15.1) Benthiazol, (15.2) Bethoxazin, (15.3) Capsimycin, (15.4) Carvon, (15.5) Chinomethionat, (15.6) Pyriofenon (Chlazafenon), (15.7) Cufraneb, (15.8) Cyflufenamid, (15.9) Cymoxanil, (15.10) Cyprosulfamid, (15.11) Dazomet, (15.12) Debacarb, (15.13) Dichlorphen, (15.14) Diclomezin, (15.15) Difenzoquat, (15.16) Difenzoquat Methylsulphat, (15.17) Diphenylamin, (15.18) Ecomat, (15.19) Fenpyrazamin, (15.20) Flumetover, (15.21) Fluorimid, (15.22) Flusulfamid, (15.23) Flutianil, (15.24) Fosetyl-Aluminium, (15.25) Fosetyl-Calcium, (15.26) Fosetyl-Natrium, (15.27) Hexachlorbenzol, (15.28) Irumamycin, (15.29) Methasulfocarb, (15.30) Methylisothiocyanat, (15.31) Metrafenon, (15.32) Mildiomycin, (15.33) Natamycin, (15.34) Nickel Dimethyldithiocarbamat, (15.35) Nitrothal-Isopropyl, (15.36) Octhilinone, (15.37) Oxamocarb, (15.38) Oxyfenthiin, (15.39) Pentachlorphenol und dessen Salze, (15.40) Phenothrin, (15.41) Phosphorsäure und deren Salze, (15.42) Propamocarb-Fosetylat, (15.43) Propanosin-Natrium, (15.44) Pyrimorph, (15.45) (2E)-3-(4-Tert-butylphenyl)-3-(2-chlorpyridin-4-yl)-1-(morpholin-4-yl)prop-2-en-1-on, (15.46) (2Z)-3-(4-Tert-butylphenyl)-3-(2-chlorpyridin-4-yl)-1-(morpholin-4-yl)prop-2-en-1-on, (15.47) Pyrrolnitrin, (15.48) Tebufloquin, (15.49) Tecloftalam, (15.50) Tolnifanide, (15.51) Triazoxid, (15.52) Trichlamid, (15.53) Zarilamid, (15.54) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, (15.55) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.56) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.57) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.58) 1-(4-Methoxyphenoxy)-3,3-dimethylbutan-2-yl 1H-imidazole-1-carboxylat, (15.59) 2,3,5,6-Tetrachlor-4-(methylsulfonyl)pyridin, (15.60) 2,3-Dibutyl-6-chlorthieno[2,3-d]pyrimidin-4(3H)-on, (15.61) 2,6-Dimethyl-1H,5H-[1,4]dithiino[2,3-c:5,6-c']dipyrrole-1,3,5,7(2H,6H)-tetron, (15.62) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.63) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.64) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.65) 2-Butoxy-6-iodo-3-propyl-4H-chromen-4-on, (15.66) 2-Chlor-5-[2-chlor-1-(2,6-difluor-4-methoxyphenyl)-4-methyl-1H-imidazol-5-yl]pyridin, (15.67) 2-Phenylphenol und Salze, (15.68) 3-(4,4,5-Trifluor-3,3-dimethyl-3,4-dihydroisochinolin-1-yl)chinolin, (15.69) 3,4,5-Trichlorpyridine-2,6-dicarbonitril, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazole-2-thiol, (15.73) 5-Chlor-N'-phenyl-N'-(prop-2-yn-1-yl)thiophene-2-sulfonohydrazid, (15.74) 5-Fluor-2-[(4-fluorbenzyl)oxy]pyrimidin-4-amin, (15.75) 5-Fluor-2-[(4-methylbenzyl)oxy]pyrimidin-4-amin, (15.76) 5-Methyl-6-octyl[1,2,4]triazolo[1,5-a]pyrimidin-7-amin, (15.77) Ethyl (2Z)-3-amino-2-cyano-3-phenylacrylat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-yn-1-yloxy)phenyl]propanamid, (15.80) N-[(4-Chlorphenyl)(cyano)methyl]-3-[3-methoxy-4-(prop-2-yn-1-yloxy)phenyl]propanamid, (15.81) N-[(5-Brom-3-chlorpyridin-2-yl)methyl]-2,4-dichlornicotinamid, (15.82) N-[1-(5-Brom-3-chlorpyridin-2-yl)ethyl]-2,4-dichlornicotinamid, (15.83) N-[1-(5-Brom-3-chlorpyridin-2-yl)ethyl]-2-fluor-4-iodonicotinamid, (15.84) N-{(E)-[(Cyclopropylmethoxy)immo][6-(difluormethoxy)-2,3-difluorphenyl]methyl}-2-phenyl-acetamid, (15.85) N-{(Z)-[(Cyclopropylmethoxy)imino][6-(difluormethoxy)-2,3-difluorphenyl]methyl}-2-phenylacetamid, (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazole-4-carboxamid, (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazole-4-carboxamid, (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazole-4-carboxamid, (15.90) Pentyl{6-[({[(1-methyl-1H-tetrazol-5-yl)(phenyl)methylene]amino}oxy)methyl]pyridin-2-yl}carbamat, (15.91) Phenazine-1 -carbonsäure, (15.92) Chinolin-8-ol, (15.93) Chinolin-8-ol sulfate (2:1), (15.94) Tert-butyl {6-[({[(1-methyl-1H-tetrazol-5-yl)(phenyl)methylene]amino}oxy)methyl]pyridin-2-yl}carbamat, (15.95) 1-Methyl-3-(trifluormethyl)-N-[2'-(trifluormethyl)biphenyl-2-yl]-1H-pyrazol-4-carboxamid, (15.96) N-(4'-Chlorbiphenyl-2-yl)-3-(difluormethyl)-1-methyl-1H-pyrazol-4-carboxamid, (15.97) N-(2',4'-Dichlorbiphenyl-2-yl)-3-(difluormethyl)-1-methyl-1H-pyrazol-4-carboxamid, (15.98) 3-(Difluormethyl)-1-methyl-N-[4'-(trifluormethyl)biphenyl-2-yl]-1H-pyrazol-4-carboxamid, (15.99) N-(2',5'-Difluorbiphenyl-2-yl)-1-methyl-3-(trifluormethyl)-1H-pyrazol-4-carboxamid, (15.100) 3-(Difluormethyl)-1-methyl-N-[4'-(prop-1-yn-1-yl)biphenyl-2-yl]-1H-pyrazol-4-carboxamid, (15.101) 5-Fluor-1,3-dimethyl-N-[4'-(prop-1-yn-1-yl)biphenyl-2-yl]-1H-pyrazol-4-carboxamid, (15.102) 2-Chlor-N-[4'-(prop-1-yn-1-yl)biphenyl-2-yl]nicotinamid, (15.103) 3-(Difluormethyl)-N-[4'-(3,3-dimethylbut-1-yn-1-yl)biphenyl-2-yl]-1-methyl-1H-pyrazol-4-carboxamid, (15.104) N-[4'-(3,3-dimethylbut-1-yn-1-yl)biphenyl-2-yl]-5-fluor-1,3-dimethyl-1H-pyrazol-4-carboxamid, (15.105) 3-(Difluormethyl)-N-(4'-ethynylbiphenyl-2-yl)-1-methyl-1H-pyrazol-4-carboxamid, (15.106) N-(4'-Ethynylbiphenyl-2-yl)-5-fluor-1,3-dimethyl-1H-pyrazol-4-carboxamid, (15.107) 2-Chlor-N-(4'-ethynylbiphenyl-2-yl)nicotinamid, (15.108) 2-Chlor-N-[4'-(3,3-dimethylbut-1-yn-1-yl)biphenyl-2-yl]nicotinamid, (15.109) 4-(Difluormethyl)-2-methyl-N-[4'-(trifluormethyl)biphenyl-2-yl]-1,3-thiazole-5-carboxamid, (15.110) 5-Fluor-N-[4'-(3-hydroxy-3-methylbut-1-yn-1-yl)biphenyl-2-yl]-1,3-dimethyl-1H-pyrazol-4-carboxamid, (15.111) 2-Chlor-N-[4'-(3-hydroxy-3-methylbut-1-yn-1-yl)biphenyl-2-yl]nicotinamid, (15.112) 3-(Difluormethyl)-N-[4'-(3-methoxy-3-methylbut-1-yn-1-yl)biphenyl-2-yl]-1-methyl-1H-pyrazol-4-carboxamid, (15.113) 5-Fluor-N-[4'-(3-methoxy-3-methylbut-1-yn-1-yl)biphenyl-2-yl]-1,3-dimethyl-1H-pyrazol-4-carboxamid, (15.114) 2-Chlor-N-[4'-(3-methoxy-3-methylbut-1-yn-1-yl)biphenyl-2-yl]nicotinamid, (15.115) (5-Brom-2-methoxy-4-methylpyridin-3-yl)(2,3,4-trimethoxy-6-methylphenyl)methanon, (15.116) N-[2-(4-{[3-(4-Chlorphenyl)prop-2-yn-1-yl]oxy}-3-methoxyphenyl)ethyl]-N2-(methylsulfonyl)valinamid, (15.117) 4-Oxo-4-[(2-phenylethyl)amino]butansäure, (15.118) But-3-yn-1-yl {6-[({[(Z)-(1-methyl-1H-tetrazol-5-yl)(phenyl)methylene]amino}oxy)methyl]pyridin-2-yl}carbamat, (15.119) 4-Amino-5-fluorpyrimidin-2-ol (Tautomere Form: 4-Amino-5-fluorpyrimidin-2(1H)-on), (15.120) Propyl 3,4,5-trihydroxybenzoat, (15.121) 1,3-Dimethyl-N-(1,1,3-trimethyl-2,3-dihydro-1H-inden-4-yl)-1H-pyrazol-4-carboxamid, (15.122) 1,3-Dimethyl-N-[(3R)-1,1,3-trimethyl-2,3-dihydro-1H-inden-4-yl]-1H-pyrazol-4-carboxamid, (15.123) 1,3-Dimethyl-N-[(3S)-1,1,3-trimethyl-2,3-dihydro-1H-inden-4-yl]-1H-pyrazol-4-carboxamid, (15.124) [3-(4-Chlor-2-fluorphenyl)-5-(2,4-difluorphenyl)-1,2-oxazol-4-yl](pyridin-3-yl)methanol, (15.125) (S)-[3-(4-Chlor-2-fluorphenyl)-5-(2,4-difluorphenyl)-1,2-oxazol-4-yl](pyridin-3-yl)methanol, (15.126) (R)-[3-(4-Chlor-2-fluorphenyl)-5-(2,4-difluorphenyl)-1,2-oxazol-4-yl](pyridin-3-yl)methanol, (15.127) 2-{[3-(2-Chlorphenyl)-2-(2,4-difluorphenyl)oxiran-2-yl]methyl}-2,4-dihydro-3H-1,2,4-triazol-3-thion, (15.128) 1-{[3-(2-Chlorphenyl)-2-(2,4-difluorphenyl)oxiran-2-yl]methyl}-1H-1,2,4-triazol-5-yl thiocyanat, (15.129) 5-(Allylsulfanyl)-1-{[3-(2-chlorphenyl)-2-(2,4-difluorphenyl)oxiran-2-yl]methyl}-1H-1,2,4-triazol, (15.130) 2-[1-(2,4-Dichlorphenyl)-5-hydroxy-2,6,6-trimethylheptan-4-yl]-2,4-dihydro-3H-1,2,4-triazol-3-thion, (15.131) 2-{[rel(2R,3S)-3-(2-Chlorphenyl)-2-(2,4-difluorphenyl)oxiran-2-yl]methyl}-2,4-dihydro-3H-1,2,4-triazol-3-thion, (15.132) 2-{[rel(2R,3R)-3-(2-Chlorphenyl)-2-(2,4-difluorphenyl)oxiran-2-yl]methyl}-2,4-dihydro-3H-1,2,4-triazol-3-thion, (15.133) 1-{[rel(2R,3S)-3-(2-Chlorphenyl)-2-(2,4-difluorphenyl)oxiran-2-yl]methyl}-1H-1,2,4-triazol-5-yl thiocyanat, (15.134) 1-{[rel(2R,3R)-3-(2-Chlorphenyl)-2-(2,4-difluorphenyl)oxiran-2-yl]methyl}-1H-1,2,4-triazol-5-yl thiocyanat, (15.135) 5-(Allylsulfanyl)-1-{[rel(2R,3S)-3-(2-chlorphenyl)-2-(2,4-difluorphenyl)oxiran-2-yl]methyl}-1H-1,2,4-triazol, (15.136) 5-(Allylsulfanyl)-1-{[rel(2R,3R)-3-(2-chlorphenyl)-2-(2,4-difluorphenyl)oxiran-2-yl]methyl}-1H-1,2,4-triazol, (15.137) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (15.138) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (15.139) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (15.140) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (15.141) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (15.142) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (15.143) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (15.144) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (15.145) 2-Fluor-6-(trifluormethyl)-N-(1,1,3-trimethyl-2,3-dihydro-1H-inden-4-yl)benzamid, (15.146) 2-(6-Benzylpyridin-2-yl)quinazolin, (15.147) 2-[6-(3-Fluor-4-methoxyphenyl)-5-methylpyridin-2-yl]quinazolin, (15.148) 3-(4,4-Difluor-3,3-dimethyl-3,4-dihydroisochinolin-1-yl)chinolin, (15.149) Abscisinsäure, (15.150) 3-(Difluormethyl)-N-methoxy-1-methyl-N-[1-(2,4,6-trichlorphenyl)propan-2-yl]-1H-pyrazol-4-carboxamid, (15.151) N'-[5-Brom-6-(2,3-dihydro-1H-inden-2-yloxy)-2-methylpyridin-3-yl]-N-ethyl-N-methylimidoformamid, (15.152) N'-{5-Brom-6-[1-(3,5-difluorphenyl)ethoxy]-2-methylpyridin-3-yl}-N-ethyl-N-methylimidoformamid, (15.153) N'-{5-Brom-6-[(1R)-1-(3,5-difluorphenyl)ethoxy]-2-methylpyridm-3-yl}-N-ethyl-N-methylimidoformamid, (15.154) N'-{5-Brom-6-[(1S)-1-(3,5-difluorphenyl)ethoxy]-2-methylpyridin-3-yl}-N-ethyl-N-methylimidoformamid, (15.155) N'- {5-Brom-6-[(cis-4-isopropylcyclohexyl)oxy]-2-methylpyridin-3-yl} -N-ethyl-N-methylimidoformamid, (15.156) N'-{5-Brom-6-[(trans-4-isopropyl-cyclohexyl)oxy]-2-methylpyridin-3-yl} -N-ethyl-N-methylimidoformamid, (15.157) N-Cyclopropyl-3-(difluormethyl)-5-fluor-N-(2-isopropylbenzyl)-1-methyl-1H-pyrazol-4-carboxamid, (15.158) N-Cyclopropyl-N-(2-cyclopropylbenzyl)-3-(difluormethyl)-5-fluor-1-methyl-1H-pyrazol-4-carboxamid, (15.159) N-(2-Tert-butylbenzyl)-N-cyclopropyl-3-(difluormethyl)-5-fluor-1-methyl-1H-pyrazol-4-carboxamid, (15.160) N-(5-Chlor-2-ethylbenzyl)-N-cyclopropyl-3-(difluormethyl)-5-fluor-1-methyl-1H-pyrazol-4-carboxamid, (15.161) N-(5-Chlor-2-isopropylbenzyl)-N-cyclopropyl-3-(difluormethyl)-5-fluor-1-methyl-1H-pyrazol-4-carboxamid, (15.162) N-Cyclopropyl-3-(difluormethyl)-N-(2-ethyl-5-fluorbenzyl)-5-fluor-1-methyl-1H-pyrazol-4-carboxamid, (15.163) N-Cyclopropyl-3-(difluormethyl)-5-fluor-N-(5-fluor-2-isopropylbenzyl)-1-methyl-1H-pyrazol-4-carboxamid, (15.164) N-Cyclopropyl-N-(2-cyclopropyl-5-fluorbenzyl)-3-(difluormethyl)-5-fluor-1-methyl-1H-pyrazol-4-carboxamid, (15.165) N-(2-Cyclopentyl-5-fluorbenzyl)-N-cyclopropyl-3-(difluormethyl)-5-fluor-1-methyl-1H-pyrazol-4-carboxamid, (15.166) N-Cyclopropyl-3-(difluormethyl)-5-fluor-N-(2-fluor-6-isopropylbenzyl)-1-methyl-1H-pyrazol-4-carboxamid, (15.167) N-Cyclopropyl-3-(difluormethyl)-N-(2-ethyl-5-methylbenzyl)-5-fluor-1-methyl-1H-pyrazol-4-carboxamid, (15.168) N-Cyclopropyl-3-(difluormethyl)-5-fluor-N-(2-isopropyl-5-methylbenzyl)-1-methyl-1H-pyrazol-4-carboxamid, (15.169) N-Cyclopropyl-N-(2-cyclopropyl-5-methylbenzyl)-3-(difluormethyl)-5-fluor-1-methyl-1H-pyrazol-4-carboxamid, (15.170) N-(2-Tert-butyl-5-methylbenzyl)-N-cyclopropyl-3-(difluormethyl)-5-fluor-1-methyl-1H-pyrazol-4-carboxamid, (15.171) N-[5-Chlor-2-(trifluormethyl)benzyl]-N-cyclopropyl-3-(difluormethyl)-5-fluor-1-methyl-1H-pyrazol-4-carboxamid, (15.172) N-Cyclopropyl-3-(difluormethyl)-5-fluor-1-methyl-N-[5-methyl-2-(trifluormethyl)benzyl]-1H-pyrazol-4-carboxamid, (15.173) N-[2-Chlor-6-(trifluormethyl)benzyl]-N-cyclopropyl-3-(difluormethyl)-5-fluor-1-methyl-1H-pyrazol-4-carboxamid, (15.174) N-[3-Chlor-2-fluor-6-(trifluormethyl)benzyl]-N-cyclopropyl-3-(difluormethyl)-5-fluor-1-methyl-1H-pyrazol-4-carboxamid, (15.175) N-Cyclopropyl-3-(difluormethyl)-N-(2-ethyl-4,5-dimethylbenzyl)-5-fluor-1-methyl-1H-pyrazol-4-carboxamid (15.176) N-Cyclopropyl-3-(difluormethyl)-5-fluor-N-(2-isopropylbenzyl)-1-methyl-1H-pyrazol-4-carbothioamid, (15.177) 3-(Difluormethyl)-N-(7-fluor-1,1,3-trimethyl-2,3-dihydro-1H-inden-4-yl)-1-methyl-1H-pyrazol-4-carboxamid, (15.178) 3-(Difluormethyl)-N-[(3R)-7-fluor-1,1,3-trimethyl-2,3-dihydro-1H-inden-4-yl]-1-methyl-1H-pyrazol-4-carboxamid, (15.179) 3-(Difluormethyl)-N-[(3S)-7-fluor-1,1,3-trimethyl-2,3-dihydro-1H-inden-4-yl]-1-methyl-1H-pyrazol-4-carboxamid, (15.180) N'-(2,5-Dimethyl-4-phenoxyphenyl)-N-ethyl-N-methylimidoformamid, (15.181) N'-{4-[(4,5-Dichlor-1,3-thiazol-2-yl)oxy]-2,5-dimethylphenyl}-N-ethyl-N-methylimidoformamid, (15.182) N-(4-Chlor-2,6-difluorphenyl)-4-(2-chlor-4-fluorphenyl)-1,3-dimethyl-1H-pyrazol-5-amin. Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
Bacillus amyloliquefaciens, Stamm FZB42 (DSM 231179), oder Bacillus cereus, insbesondere B. cereus Stamm CNCM I-1562 oder Bacillus firmus, Stamm I-1582 (Accession number CNCM I-1582) oder Bacillus pumilus, insbesondere Stamm GB34 (Accession No. ATCC 700814) und Stamm QST2808 (Accession No. NRRL B-30087), oder Bacillus subtilis, insbesondere Stamm GB03 (Accession No. ATCC SD-1397), oder Bacillus subtilis Stamm QST713 (Accession No. NRRL B-21661) oder Bacillus subtilis Stamm OST 30002 (Accession No. NRRL B-50421) Bacillus thuringiensis, insbesondere B. thuringiensis subspecies israelensis (serotype H-14), Stamm AM65-52 (Accession No. ATCC 1276), oder B. thuringiensis subsp. aizawai, insbesondere Stamm ABTS-1857 (SD-1372), oder B. thuringiensis subsp. kurstaki Stamm HD-1, oder B. thuringiensis subsp. tenebrionis Stamm NB 176 (SD-5428), Pasteuria penetrans, Pasteuria spp. (Rotylenchulus reniformis nematode)-PR3 (Accession Number ATCC SD-5834), Streptomyces microflavus Stamm AQ6121 (= QRD 31.013, NRRL B-50550), Streptomyces galbus Stamm AQ 6047 (Acession Number NRRL 30232).

Beispiele für Pilze und Hefen, die als biologische Schädlingsbekämpfungsmittel eingesetzt werden bzw. verwendet werden können, sind:
Beauveria bassiana, insbesondere Stamm ATCC 74040, Coniothyrium minitans, insbesondere Stamm CON/M/91-8 (Accession No. DSM-9660), Lecanicillium spp., insbesondere Stamm HRO LEC 12, Lecanicillium lecanii, (ehemals bekannt als Verticillium lecanii), insbesondere Stamm KV01, Metarhizium anisopliae, insbesondere Stamm F52 (DSM3884/ ATCC 90448), Metschnikowia fructicola, insbesondere Stamm NRRL Y-30752, Paecilomyces fumosoroseus (neu: Isaria fumosorosea), insbesondere Stamm IFPC 200613, oder Stamm Apopka 97 (Accesion No. ATCC 20874), Paecilomyces lilacinus, insbesondere P. lilacinus Stamm 251 (AGAL 89/030550), Talaromyces flavus, insbesondere Stamm V117b, Trichoderma atroviride, insbesondere Stamm SC1 (Accession Number CBS 122089), Trichoderma harzianum, insbesondere T. harzianum rifai T39. (Accession Number CNCM I-952).

Beispiele für Viren, die als biologische Schädlingsbekämpfungsmittel eingesetzt werden bzw. verwendet werden können, sind:
Adoxophyes orana (Apfelschalenwickler) Granulosevirus (GV), Cydia pomonella (Apfelwickler) Granulosevirus (GV), Helicoverpa armigera (Baumwollkapselwurm) Nuklear Polyhedrosis Virus (NPV), Spodoptera exigua (Zuckerrübeneule) mNPV, Spodoptera frugiperda (Heerwurm) mNPV, Spodoptera littoralis (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
Agrobacterium spp., Azorhizobium caulinodans, Azospirillum spp., Azotobacter spp., Bradyrhizobium spp., Burkholderia spp., insbesondere Burkholderia cepacia (ehemals bekannt als Pseudomonas cepacia), Gigaspora spp., oder Gigaspora monosporum, Glomus spp., Laccaria spp., Lactobacillus buchneri, Paraglomus spp., Pisolithus tinctorus, Pseudomonas spp., Rhizobium spp., insbesondere Rhizobium trifolii, Rhizopogon spp., Scleroderma spp., Suillus spp., Streptomyces spp..

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,"Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lager-raum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollten.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die folgenden Herstellungs- und Verwendungsbeispiele illustrieren die Erfindung ohne sie zu beschränken.

### Herstellungsbeispiele

Synthese von Ethyl-[5-phenyl-1,2,4-oxadiazol-3-yl]imidoformiat (1-7), vgl. WO 2009/011850.

### Stufe 1: Methyl-N-cyanbenzolcarboximidoat

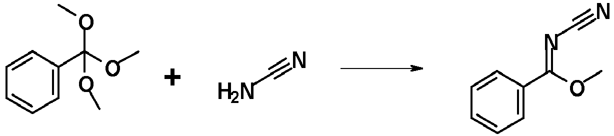

(Trimethoxymethyl)benzol (75 g) und Cyanamid (17,304 g) wurden in 78 ml Essigsäureanhydrid aufgenommen und anschließend 45 Minuten bei 135°C gerührt. Anschließend wurde im Vakuum eingeengt, der Rückstand mit Cyclohexan verrührt, der Feststoff abgesaugt und getrocknet. Es verblieben 65,30 g der Titelverbindung. HPLC-MS: log P (neutral) = 1,83; Masse (m/z): 161,1 (M+H)⁺; ¹H-NMR (DMSO-D₆) 4.045 (s, 3H), 7.601-7.644 (m, 2H), 7.712-7.755 (m, 1H), 7.966-7.995 (m, 2H).

### Stufe 2: 5-Phenyl-1,2,4-oxadiazol-3-amin

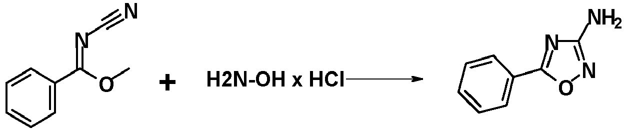

Methyl-N-cyanbenzolcarboximidoat (30,50 g) wurden in 157 ml Methanol vorgelegt und nach Zugabe von Hydroxylammoniumchlorid (13,23 g) 2 Stunden bei Raumtemperatur gerührt. Anschließend wurde bei 0°C Triethylamin (19,17 g) hinzugegeben, wobei zunächst eine klare Lösung entstand und nach 30 Minuten ein Feststoff ausfiel. Es wurde über Nacht bei Raumtemperatur gerührt, der Feststoff abgesaugt, dieser mit Wasser gewaschen und getrocknet. Es verblieben 10,60 g der Titelverbindung. HPLC-MS: log P (neutral) = 1,28; Masse (m/z): 162,1 (M+H)⁺; ¹H-NMR (DMSO-D₆) 6.418 (s, 2H), 7.514-8.02 (m, 5H).

### Stufe 3: Ethyl-[5-phenyl-1,2,4-oxadiazol-3-yl]imidoformiat

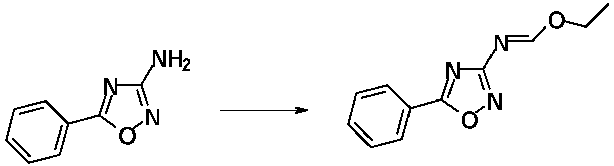

5-Phenyl-1,2,4-oxadiazol-3-amin (10,60 g) wurden mit 30 ml Orthoameisensäuretriethylester über Nacht unter Rückfluss gerührt. Anschließend ließ man abkühlen, versetzte mit 100 ml Petrolether und saugte nach 30 minütigem Rühren bei Raumtemperatur den ausgefallenen Feststoff ab. Es verblieben 9,70 g der Titelverbindung.

HPLC-MS: log P (neutral) = 2,87; Masse (m/z): 218,1 (M+H)⁺; ¹H-NMR (DMSO-D₆) 1,343 (t, 3H), 4,389 (q, 2H), 7.621-7.743 (m, 3H), 8.085-8.106 (m, 2H), 8.705 (s, 1H).

### Beispiel 1

### 2-Trifluormethyl-N-[1-(2-brom-6-fluorpheny|)-1H-1,2,4-triazol-3-yl]benzamid (Tabelle 1, Beispiel I-1-30)

### Stufe 1: N-[1-(2-Brom-6-fluor-phenyl)-1H-1,2,4-triazol-3-yl]benzamid

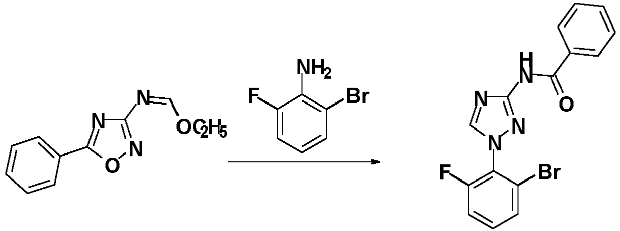

Ethyl-[5-phenyl-1,2,4-oxadiazol-3-yl]imidoformiat (2,00 g) und 2-Brom-6-fluoranilin wurden für 4 h auf 150°C erhitzt, die Reaktionsmischung auf Raumtemperatur abgekühlt, der Rückstand 45 Minuten mit 20 ml Ethanol verrührt und der zurückbleibende Feststoff abgesaugt. Zurück blieben 2,0 g der gewünschten Titelverbindung. HPLC-MS: log P (neutral) = 1,79; Masse (m/z): 361 (M+H)⁺; ¹H-NMR (DMSO-D₆) 7.587 (m, 2H), 7.638 (m, 3H), 7.768 (m, 1H), 8.002 (m, 2H), 8.891 (s, 1H), 11.053 (s, 1H).

### Stufe 2: 1-(2-Brom-6-fluor-phenyl)-1H-1,2,4-triazol-3-amin

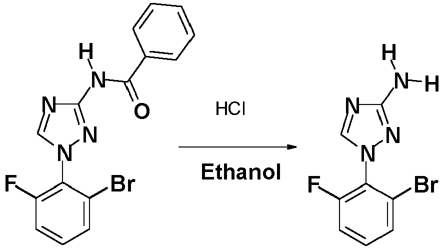

N-[1-(2-Brom-6-fluorphenyl)-1H-1,2,4-triazol-3-yl]benzamid (2,1 g) wurden in 25 ml Ethanol vorgelegt und nach Zugabe von 6 ml konzentrierter Salzsäure 8 Stunden unter Rückfluss gerührt. Anschließend wurde die Reaktionsmischung im Vakuum eingeengt, der Rückstand in Wasser aufgenommen und durch Zugabe von Natronlauge alkalisch gestellt (pH 9). Die wässrige Phase wurde mit Dichlormethan extrahiert, die vereinigten Extrakte getrocknet und eingeengt. Das verbleibende Rohprodukt wurde an Kieselgel mittels Cyclohexan/Ethylacetat als Laufmittel chromatographisch gereinigt. Nach Einengen der Extrakte verblieben 0,65 g der Titelverbindung. HPLC-MS: log P (neutral) = 0,92; Masse (m/z): 257 (M+H)⁺; ¹H-NMR (DMSO-D₆) 5.604 (s, 2H), 7.797-7.876 (m, 3H); 8.346 (s, 1H).

### Stufe 3: 2-Trifluormethyl-N-[1-(2-brom-6-fluorphenyl)-1H-1,2,4-triazol-3-yl]benzamid

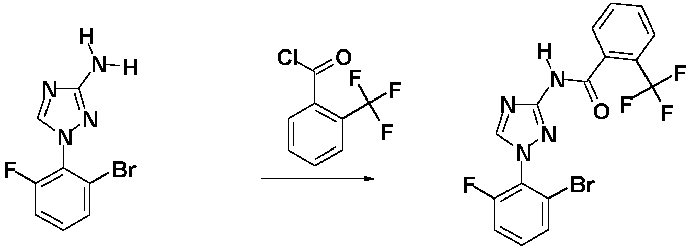

1-(2-Brom-6-fluorphenyl)-1H-1,2,4-triazol-3-amin (0,12 g) wurde zu einer Lösung von Pyridin (0,11 g) in Dichlormethan (10 ml) gegeben und bei 0 °C eine Lösung von 2-Trifluormethylbenzoylchlorid (0,107 g) in Dichlormethan zugetropft. Nach vollendeter Zugabe ließ man 4 Stunden bei 0 °C und anschließend über Nacht bei Raumtemperatur rühren. Anschließend wurde die Reaktionslösung mit weiterem Dichlormethan verdünnt, zweimal mit verdünnter Salzsäure und einmal mit Wasser gewaschen. Die organische Phase wurde abgetrennt, getrocknet und eingeengt. Der verbliebene Rückstand wurde durch Chromatographie an Kieselgel mit Cyclohexan/Ethylacetat als Laufmittel gereinigt. Es wurden 0,163 g der Titelverbindung erhalten. HPLC-MS: log P (neutral) = 2,15; Masse (m/z): 429 (M+H)⁺; ¹H-NMR (DMSO-D₆) 7.608-7.837 (m, 7H), 8.863 (s, 1H), 11.344 (s, 1H).

### Beispiel 2

### 2-Trifluormethyl-N-[1-(2,6-difluorphenyl)-1H-1,2,4-triazol-3-yl]benzamid (Tabelle 1, Beispiel I-1-11)

### Stufe 1: N-[1-(2,6-Difluor-phenyl)-1H-1,2,4-triazol-3-yl]benzamid

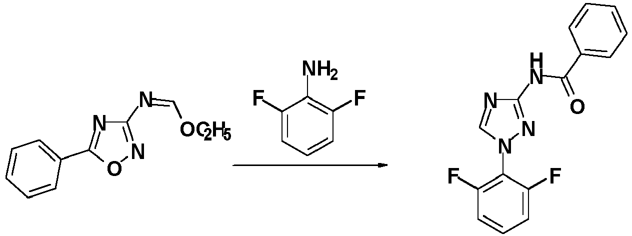

Ethyl-[5-phenyl-1,2,4-oxadiazol-3-yl]imidoformiat (5,00 g) und 2,6-Difluoranilin (2,97 g) wurden 4 Stunden bei 150 °C erhitzt und nach Abkühlen der Reaktionsmischung mit 30 ml Ethanol versetzt. Es wurde 45 Minuten bei Raumtemperatur gerührt und der zurückbleibende Feststoff abgesaugt und getrocknet. Es verblieben 6,0 g des gewünschten Produktes. HPLC-MS: log P (neutral) = 1,59; Masse (m/z): 301 (M+H)⁺; ¹H-NMR (DMSO-D₆) 7.496 (m, 4H), 7.594 (m, 1H), 7.701 (m, 1H), 7.987 (m, 2H), 8.939 (s, 1H), 11.049 (s, 1H).

### Stufe 2: 1-(2,6-Difluorphenyl)-1H-1,2,4-triazol-3-amin

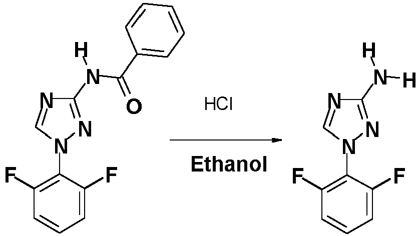

N-[1-(2,6-Difluorphenyl)-1H-1,2,4-triazol-3-yl]benzamid (1,2 g) wurden in 15 ml Ethanol vorgelegt und nach Zugabe von 3 ml konzentrierter Salzsäure 8 Stunden unter Rückfluss gerührt. Anschließend wurde die Reaktionsmischung im Vakuum eingeengt, der Rückstand in Wasser aufgenommen und durch Zugabe von Natronlauge alkalisch gestellt (pH 9). Es wurde 30 Minuten bei Raumtemperatur gerührt, der Feststoff abgesaugt, mit Diethylether verrührt und getrocknet. Zurück blieben 0,7 g der Titelverbindung. HPLC-MS: log P (neutral) = 0,61; Masse (m/z): 197,1 (M+H)⁺; ¹H-NMR (DMSO-D₆) 5.664 (s, 2H), 7.365 (m, 2H), 7.664 (m, 1H), 8.401 (s, 1H).

### 3. Stufe: 2-Trifluormethyl-N-[1-(2,6-difluorphenyl)-1H-1,2,4-triazol-3-yl]benzamid

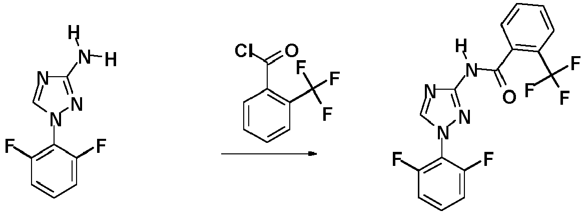

1-(2,6-Difluorphenyl)-1H-1,2,4-triazol-3-amin (1,00 g) wurde zu einer Lösung von Pyridin (2,67 g) in Dichlormethan (60 ml) gegeben und bei 0 °C - 5 °C eine Lösung von 2-Trifluormethylbenzoylchlorid (2,585 g) in Dichlormethan (15 ml) innerhalb von 20 Minuten zugetropft. Nach vollendeter Zugabe ließ man 3 Stunden bei 0 °C - 5 °C rühren. Anschließend wurde die Reaktionslösung mit weiterem Dichlormethan (60 ml) verdünnt, zweimal mit verdünnter Salzsäure (50 ml) und einmal mit Wasser gewaschen. Die organische Phase wurde abgetrennt, getrocknet und eingeengt. Der verbliebene Rückstand wurde durch Chromatographie an Kieselgel mit Cyclohexan/Ethylacetat als Laufmittel gereinigt. Es wurden 3,00 g der Titelverbindung erhalten. HPLC-MS: log P = 1,97; Masse (m/z): 369,1 (M+H)⁺; ¹H-NMR (DMSO-D₆) 7.458 (m, 2H), 7.714-7.843 (m, 5H), 8.906 (s, 1H), 11.360 (s, 1H).

### Beispiel 3:

### 2-Ethyl-N-[1-(2-brom-6-fluorphenyl)-1H-1,2,4-triazol-3-yl]benzamid (Tabelle 1, Beispiel I-1-15)

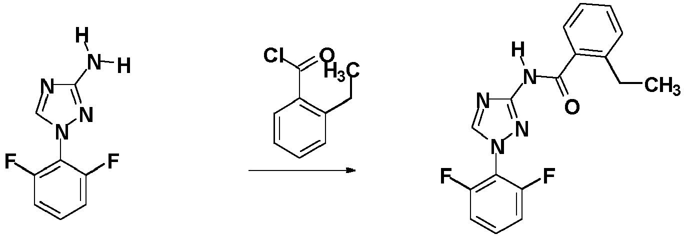

1-(2,6-Difluorphenyl)-1H-1,2,4-triazol-3-amin (0,12 g) wurde zu einer Lösung von Pyridin (0,145 g) in Dichlormethan (6 ml) gegeben und bei 0 °C - 5 °C eine Lösung von 2-Ethylbenzoylchlorid (0,113 g) in Dichlormethan (15 ml) innerhalb von 20 Minuten zugetropft. Nach vollendeter Zugabe ließ man 3 Stunden bei 0 °C - 5 °C und dann über Nacht bei Raumtemperatur rühren. Anschließend wurde die Reaktionslösung mit weiterem Dichlormethan verdünnt, zweimal mit verdünnter Salzsäure und einmal mit Wasser gewaschen. Die organische Phase wurde abgetrennt, getrocknet und eingeengt. Der verbliebene Rückstand wurde durch Chromatographie an Kieselgel mit Cyclohexan/Ethylacetat als Laufmittel gereinigt. Es wurden 0,102 g der Titelverbindung erhalten. HPLC-MS: log P (neutral) = 2,05; Masse 329,1 (m/z): (M+H)⁺; ¹H-NMR (DMSO-D₆) 1.177 (tr, 3H), 2.747 (q, 2H), 7.264-7.337 (m, 2H), 7.398-7.479 (m, 4H), 7.676-7.751 (m, 1H), 8.887 (s, 1H), 11.002 (s, 1H).

### Beispiel 4

### N-{1-[2-Fluor-6-(trifluormethyl)phenyl]-1H-1,2,4-triazol-3-yl}-2-iodobenzamid (Tabelle 1, Beispiel I-1-28)

### Stufe 1: N-{1-[2-fluor-6-(trifluormethyl)phenyl]-1H-1,2,4-triazol-3-yl}benzamid

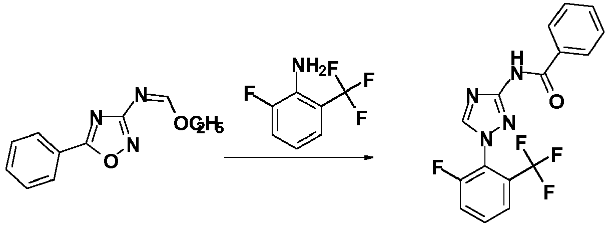

Ethyl-[5-phenyl-1,2,4-oxadiazol-3-yl]imidoformiat (2,50 g) und 2-Fluor-6-trifluormethylanilin (2,061 g) wurden über Nacht bei 150 °C gerührt und nach Abkühlen der Reaktionsmischung mit 20 ml Ethanol versetzt. Es wurde weitere 45 Minuten bei Raumtemperatur gerührt und der zurückbleibende Feststoff abgesaugt und getrocknet. Das Rohprodukt wurde an Kieselgel mit Cyclohexan/Ethylacetat als Laufmittel chromatographisch gereingt. Nach Einengen der Extrakte verblieben 1,1 g der Titelverbindung. HPLC-MS: log P (neutral) = 1,95; Masse (m/z): 351 (M+H)⁺; ¹H-NMR (DMSO-D₆) 7.501 (m, 2H), 7.601 (m, 1H), 7.883-7.984 (m, 5H), 8.893 (s, 1H), 11.038 (s, 1H).

### Stufe 2: 1-[2-Fluor-6-(trifluormethyl)phenyl]-1H-1,2,4-triazol-3-amin

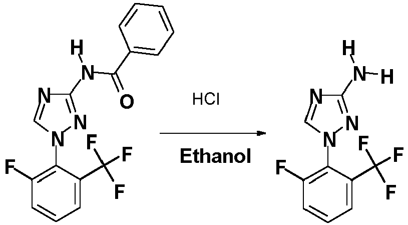

N-{1-[2-Fluor-6-(trifluormethyl)phenyl]-1H-1,2,4-triazol-3-yl}benzamid (1,2 g) wurden in 20 ml Ethanol vorgelegt und nach Zugabe von 5 ml konzentrierter Salzsäure 8 Stunden unter Rückfluss gerührt. Anschließend wurde die Raktionsmischung im Vakuum eingeengt, der Rückstand in Wasser aufgenommen und durch Zugabe von Natronlauge alkalisch gestellt (pH 9). Es wurde weitere 30 Minuten bei Raumtemperatur gerührt und der Feststoff abgesaugt und getrocknet. Zurück blieben 0,57 g der Titelverbindung. HPLC-MS: log P (neutral) = 1,09; Reinheit = 99,3 %; Masse (m/z): 247,1 (M+H)⁺; ¹H-NMR (DMSO-D₆) 5.638 (s, 2H), 7.797-7.876 (m, 3H), 8.346 (s, 1H).

### Stufe 3: N-{1-[2-Fluor-6-(trifluormethyl)phenyl]-1H-1,2,4-triazol-3-yl}-2-iodobenzamid

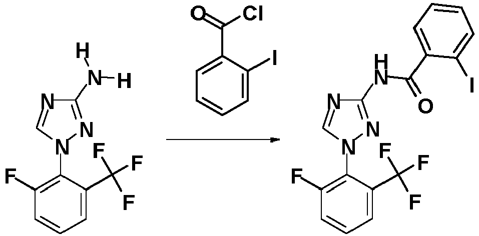

Pyridin (0,116 g) wurde in Dichlormethan (10 ml) vorgelegt und nach Zugabe von 1-[2-Fluor-6-(trifluormethyl)phenyl]-1H-1,2,4-triazol-3-amin (0,120 g) bei 0 °C 2-Iodbenzoylchlorid (0,143 g) zugegeben. Anschließend wurde zunächst 3 Stunden bei 0 °C und nachfolgend über Nacht bei Raumtemperatur gerührt. Nach Verdünnen der Reaktionsmischung mit Dichlormethan wurde die organische Phase mit verdünnter Salzsäure gewaschen, die organische Phase abgetrennt, getrocknet und im Vakuum eingeengt. Das Rohprodukt wurde an Kieselgel mit Cyclohexan/Ethylacetat als Laufmittel chromatographisch gereinigt. Nach Einengen der Extrakte verblieben 0,12 g der Titelverbindung. HPLC-MS: log P (neutral) = 2,21; Masse (m/z): 477 (M+H)⁺; ¹H-NMR (DMSO-D₆) 7.199-7.218 (m, 1H), 7.472 (m, 2H), 7.892-7.953 (m, 4H), 8.843 (s, 1H), 11.172 (s, 1H).

**Beispiel 5:**

### N-[1-(3,5-Difluorpyridin-2-yl)-1H-1,2,4-triazol-3-yl]-2-(trifluormethyl)benzamid

### Stufe 1: N-[1-(3,5-Difluorpyridin-2-yl)-1H-1,2,4-triazol-3-yl]benzamid

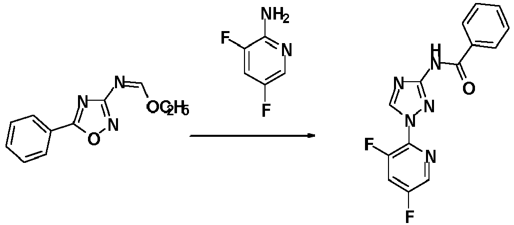

Ethyl-[5-phenyl-1,2,4-oxadiazol-3-yl]imidoformiat (2,50 g) und 3,5-Difluorpyridin-2-amin (1,497 g) wurden 4 Stunden bei 150 °C erhitzt und nach Abkühlen der Reaktionsmischung mit 10 ml Ethanol versetzt. Es wurde 45 Minuten bei Raumtemperatur gerührt und der zurückbleibende Feststoff abgesaugt und getrocknet. Es verblieben 2,50 g des gewünschten Produktes HPLC-MS: log P (neutral) = 1,36; Masse (m/z): 302,1 (M+H)⁺; ¹H-NMR (DMSO-D₆) 7.514-7.577 (m, 2H), 7.596-7.636 (m, 1H), 7.997 (m, 2H), 8.359-8.412 (m, 1H), 8.587 (d, 1H), 9.103 (s, 1H) 11.062 (s, 1H).

### Stufe 2: 1-(3,5-Difluorpyridin-2-yl)-1H-1,2,4-triazol-3-amin

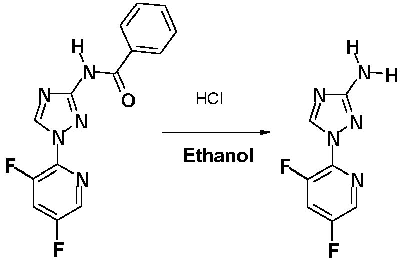

N-[1-(3,5-Difluorpyridin-2-yl)-1H-1,2,4-triazol-3-yl]benzamid (2,4 g) wurden in 25 ml Ethanol vorgelegt und nach Zugabe von 4 ml konzentrierter Salzsäure 8 Stunden unter Rückfluss gerührt. Anschließend wurde die Reaktionsmischung im Vakuum eingeengt, der Rückstand in Wasser aufgenommen und durch Zugabe von Natronlauge alkalisch gestellt (pH 8). Es wurde 30 Minuten bei Raumtemperatur gerührt, der Feststoff abgesaugt und getrocknet. Zurück blieben 0,130 g der Titelverbindung. HPLC-MS: log P = 0,30; Masse (m/z): 198 (M+H)⁺; ¹H-NMR (DMSO-D₆) 5.808 (s, 2H), 8,219-8.273 (m, 1H), 8,455 (d, 1H), 8.663 (s, 1H).

### Stufe 3: N-[1-(3,5-Difluorpyridin-2-yl)-1H-1,2,4-triazol-3-yl]-2-(trifluormethyl)benzamid

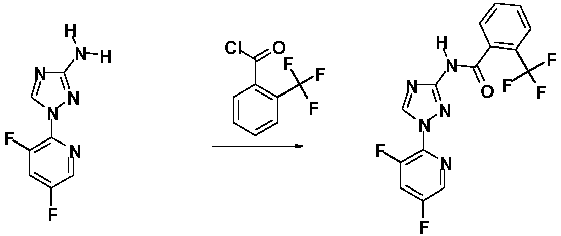

Zu 1-(3,5-Difluorpyridin-2-yl)-1H-1,2,4-triazol-3-amin (0,10 g) in Pyridin (2,934 g) wurde bei 0 °C 2-Trifluormethylbenzoylchlorid (0,116 g) gegeben. Nach vollendeter Zugabe ließ man über Nacht bei Raumtemperatur rühren, verrührte die Reaktionsmischung mit verdünnter Salzsäure, filtrierte den zurückbleibenden Feststoff ab und wusch diesen mit Wasser. Nach dem Trocknen verblieben 0,150 g der gewünschten Titelverbindung. HPLC-MS: log P = 1,76; Masse (m/z): 370 (M+H)⁺; ¹H-NMR (DMSO-D₆) 7.706-7.847 (m, 4H), 8.377(m, 1H), 8.575 (s, 1H), 9.058 (s, 1H), 11.369 (s, 1H).

Die folgenden Verbindungen der Formel (IIa-1), in denen R3 für Wasserstoff steht, wurden analog zu den angegebenen Beispielen erhalten:

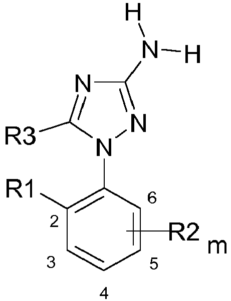

| **R1** | **R2ₘ** | **logP (neutral)** | **¹H-NMR (DMSO-D₆)** |
|---|---|---|---|
| F | - | 0,86 | 8.5 (s, 1H), 7.709 (m, 1H), 7.483 (m, 1H), 7.385 (m, 2H), 5.749 (s, 2H) |
| Cl | - | 0,96 | 5.624 (s, 2H), 7.680 (m, 1H), 7.497 (m, 2H), 7.592 (m, 2H), 8.438 (s, 2H) |
| CF₃ | - | 1,16 | 8.279 (s, 1H), 7.937 (m, 1H), 7,858 (m, 1H), 7.737 (m, 1H), 7.641 (m, 2H), 5.578 (s, 2H) |
| OCF₃ | - | 1,44 | 8.437 (s, 1H), 7.740 (m, 1H),7.493 - 7.588 (m, 4H), 5.707 (s, 2H) |
| Br | - | 1,03 | 8.387 (s, 1H), 7,828 (m, 1H), 7.530 (m, 1H), 7.383 (m, 1H), 5.587 (s, 2H) |
| I | - | 1,13 | 8.314 (s, 1H), 8,002 (m, 1H), 7.544 (m, 1H), 7.447 (m, 1H), 7.259 (m, 1H), 5.539 (s, 2H) |
| Cl | 6-F | 0,86 | 8.365 (s, 1H), 7.475 - 7.602 (m, 3H), 5.623 (s, 2H) |
| Br | 6-Br | 1,2 | 8.291 (s, 1H), 7.865 (m, 2H), 7.433 (m, 1H), 5.564 (s, 2H) |
| F | 6-I | 1,07 | 8.309 (s, 1H9, 7.839 (m, 1H), 7.512 (m, 1H), 7.381 (m, 1H), 5.580 (s, 2H) |
| F | 6-CH₃ | 1,03 | 8.271 (s, 1H), 7.408 (m, 1H), 7.287 (m, 1H),7.175 (m, 1H), 5.510 (s, 2H), 2.237 (s, 3H) |
| Cl | 6-CH₃ | 1,12 | 8.228 (s, 1H), 7.509 (m, 1H);7.419 (m, 1H), 7.419 (m, 1H), 5.517 (s, 2H), 2.498 (s, 3H) |
| Cl | 6-C₂H₅ | 1,12 | 8.249 (s, 1H), 7.523 (m, 2H), 7.401 (m, 1H), 5.512 (s, 2H), 2.397 (q, 2H), 1.036 (t, 3H) |
| Cl | 6-Cl | 0,94 | 8.327 (s, 1H), 7.690 (m, 2H), 7.548 - 7.585 (m, 1H), 5.593 (s, 2H) |
| CH₃ | 6-OCH₃ | 0,98 | 8.039 (s, 1H), 7.348 (m, 1H), 7.029 (m, 1H), 6.948 (m, 1H); 5.364 (s, 2H), 3.718 (s, 3H), 2.043 (s, 3H) |
| Cl | 3-Cl | 1,41 | 8.483 (s, 1H), 7.756 (m, 1H), 7.588 (m, 1H), 7.492 (m, 1H), 5.681 (s, 2H) |
| Cl | 4-Cl | 1,48 | 8.461 (s, 1H), 7.885 (m, 1H), 7.587 (m, 2H), 5,677 (s, 2H) |
| Cl | 5-Cl | 1,44 | 8.515 (s, 1H),7.713 (m, 2H), 7.526 (m, 1H), 5.717 (s, 2H) |
| F | 3,6-F₂ | 0,84 | 8.455 (s, 1H), 7.710 (m, 1H), 7.454 (m, 1H), 5.761 (s, 2H) |
| F | 4,6-F₂ | 0,82 | 8.379 (s, 1H), 7.521 (m, 2H), 5.675 (s, 2H) |
| F | 3,4,5,6-F₄ | 1,31 | 8.484 (s, 1H), 5.868 (s, 2H) |
| Br | 6-Br-4-F | 1,35 | 8.276 (s, 1H), 7.939 (m, 2H), 5.575 (s, 2H) |

Die folgenden N-[1-Aryl-1H-1,2,4-triazol-3-yl]benzamide (vgl. Formel (1-9) im Syntheseschema 1) wurden analog zu den angegebenen Beispielen erhalten:

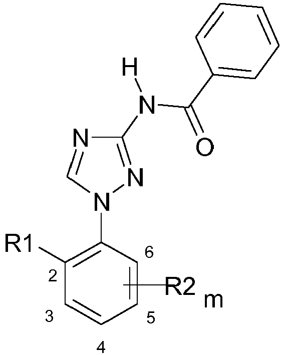

| **R1** | **R2ₘ** | **logP (neutral)** | **¹H-NMR (DMSO-D₆)** |
|---|---|---|---|
| F | - | 1,69 | 11.030 (s, 1H), 8.962 (s, 1H), 8.015 (m, 2H), 7.842 (m, 1H), 7.588 (m, 5H), 7.449 (m, 1H) |
| Cl | - | 1,75 | 10.999 (s, 1H), 8.906 (s, 1H), 8.006 (m, 2H), 7.776 (m, 1H), 7.719 (m, 1H), 7.694 (m, 3H), 7.523 (m, 2H) |
| CF₃ | - | 1,91 | 10.985 (s, 1H), 8.819 (s, 1H), 7.756 - 7,991 (m, 6H), 7.610 (m, 1H), 7.520 (m, 2H) |
| OCF₃ | - | 2,15 | 11.018 (s, 1H), 8,920 (s, 1H), 8.011 (m, 2H), 7.828 (m, 1H), 7.697 (m, 4H) 7.5088 (m, 2H) |
| Br | - | 1,80 | 11.001 (s, 1H), 8.876 (s, 1H), 7.987 (m, 2H), 7.899 (m, 1H), 7.504 - 7.679 (m, 6H) |
| I | - | 1,87 | 10.994 (s, 1H), 8.824 (s, 1H), 7.988 - 8.098 (m, 3H), 7.585 - 7.604 (m, 4H), 7.364 (m, 1H) |
| Cl | 6-F | 1,75 | 11.050 (s, 1H), 8.907 (s, 1H), 8.002 (m, 2H), 7.572 - 7.710 (m, 4H), 7.504 (m, 2H) |
| F | 6-CH₃ | 1,89 | 10.924 (s, 1H), 8.778 (s, 1H), 8.001 (m, 2H), 7.605 (m, 1H), 7.503 (m, 3H), 7.380 (m, 1H), 7.269 (m, 1H), 2.247 (s, 3H) |
| F | 6-I | 1,79 | 11.047 (s, 1H), 8.857 (s, 1H), 7.989 (m, 3H), 7.424 - 7.604 (m, 5H) |
| Cl | 6-CH₃ | 1,92 | 10.990 (s, 1H), 8.781 (s, 1H) 8.004 (m, 2H), 7.579 (m, 2H), 7.501 (m, 4H), 2.111 (s, 3H) |
| Cl | 6-C₂H₅ | 2,2 | 10.977 (s, 1H), 8.812 (s, 1H), 8.004 (m, 2H), 7.489 - 7.592 (m, 6H), 2.383 (q, 2H), 1,061 (t, 3H) |
| Cl | 6-Cl | 1,8 | 11.062 (s, 1H),8.875 (s, 1H), 7.985 (m, 2H), 7.780 (m, 7H), 7.500 - 7.701 (m, 4H) |
| Br | 6-Br | 2,00 | 11.075 (s, 1H), 8.845 (s, 1H), 8.203 (m, 1H), 7.823 - 8.036 (m, 3H), 7.499 - 7.695 (m, 4H) |
| CH₃ | 6-OCH₃ | 1,76 | 10.861 (s, 1H), 8.584 (s, 1H), 7.997 (m, 2H), 7.616 (m, 1H), 7.516 (m, 1H), 7.498 (m, 1H), 7.111 (m, 1H), 7.004 (m, 1H), 3.758 (s, 3H), 2.060 (s, 3H) |
| Cl | 3-Cl | 2,11 | 11.043 (s, 1H), 8.939 (s, 1H), 7.985 (m, 2H), 7.505 - 7,627 (m, 6H) |
| Cl | 4-Cl | 2,18 | 11.031 (s, 1H), 8.912 (s, 1H), 8.002 (m, 3H), 7.750 (m, 2H), 7.551 (m, 3H) |
| Cl | 5-Cl | 2,17 | 11.059 (s, 1H), 8.942 (s, 1H), 8.005 (m, 2H), 7.987 (m, 1H), 7.794 (m, 1H), 7.703 (m, 1H), 7.628 8m, 1H), 7.507 (m, 2H) |
| F | 3,6-F₂ | 1,73 | 11.102 (s, 1H), 8.971 (s, 1H), 7.898 (m, 3H), 7.805 (m, 1H), 7.560 (m, 1H), 7.510 (m, 3H) |
| F | 4,6-F₂ | 1,71 | 11.056 (s, 1H), 8.911 (s, 1H), 7.981 (m, 2H), 7.611 (m, 3H), 7.506 (m, 2H) |
| Br | 6-Br-4-F | 2,14 | 11.088 (s, 1H), 8.827 (s, 1H), 8.205 (m, 1H), 8.001 (m, 3H), 7.733 (m, 1H), 7.582 (m, 3H) |
| F | 3,4,5,6-F₄ | 2,11 | 11.163 (s, 1H), 8.991 (s, 1H), 7.986 (m, 2H), 7.528 - 7.652 (m, 3H) |

Ausgehend von geeignet substituierten 3-Amino-5-aryl-1,2,4-oxadiazolen, die mit Hilfe literaturbekannter Methoden herstellbar sind (Heterocycles, 57, 811-823), lassen sich die gewünschten Verbindungen der Formel (I-1) direkt durch Umsetzung entsprechend substituierter Alkoxy-[5-aryl-1,2,4-oxadiazol-3-yl-]imidoformiate (2-8) mit geeigneten Anilinen herstellen.

### Beispiel 6:

### 2-Chlor-N-[1-(2,6-difluorphenyl)-1H-1,2,4-triazol-3-yl]benzamid

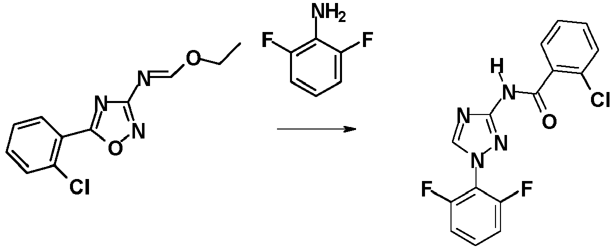

Ethyl [5-(2-chlorophenyl)-1,2,4-oxadiazol-3-yl]imidoformiat (0,1g) und 2,6-Difluoranilin (0, 05 g) wurden 30 Minuten bei 170 °C und 250 Watt in einer Mikrowellenapparatur gerührt. Der verbliebene Rückstand wurde an Kieselgel mittels Cyclohexan/Ethylacetat als Laufmittel chromatographisch gereinigt. Nach Einengen der Extrakte verblieben 0,017 g der Titelverbindung. HPLC-MS: log P (neutral) = 1,73; Masse (m/z): 257 (M+H)⁺; ¹H-NMR (DMSO-D₆) 7.416-7.750 (m, 7H); 8.891 (s, 1H); 11.272 (s, 1H).

### Beispiel 7:

### N-[2-(2-Methoxyphenyl)-2H-tetrazol-5-yl]-2-(trifluormethyl)benzamid (Tabelle 2, Beispiel I-2-1)

### Stufe 1: N-(2H-Tetrazol-5-yl)-2-(trifluormethyl)benzamid

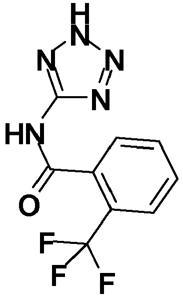

2H-Tetrazol-5-amin Hydrat (200 mg) wurde in N, N-Dimethylformamid (1 mL) vorgelegt, bei 0 °C mit Triethylamin (294 mg) versetzt und 1 Stunde bei 0 °C gerührt. Anschließend wurde 2-(Trifluormethyl)benzoylchlorid (607 mg) bei 0 °C zugetropft, weitere 2 Stunden bei 0 °C gerührt und auf Raumtemperatur erwärmt. Ein farbloser Niederschlag präzipitierte, er wurde abfiltriert und getrocknet. Es wurden 73 mg der Titelverbindung erhalten. Die Mutterlauge wurde mit Wasser/1% Ameisensäure/Acetonitril als Laufmittel säulenchromatographisch (Gradient = 30 min von 95 % Wasser/1% Ameisensäure auf 100 % Acetonitril) gereinigt. So wurden weitere 110 mg der Titelverbindung erhalten. HPLC-MS: logP = 1,17; Masse (m/z): 258,1 (M+H)⁺; ¹H-NMR (DMSO-D₆) 7.71 - 7.93 (m, 4H), 12.6 (s, 1H), 16.2 (s, 1H).

### Stufe 2: N-[2-(2-Methoxyphenyl)-2H-tetrazol-5-yl]-2-(trifluormethyl)benzamid

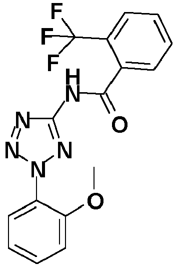

(2-Methoxyphenyl)boronsäure (144 mg) wurde in N,N-Dimethylformamid (1,5 mL) vorgelegt, anschließend nacheinander mit N-(2H-Tetrazol-5-yl)-2-(trifluormethyl)benzamid (122 mg), Kupfer(II)acetat (129 mg), Pyridin (75 mg) und Molekularsieb 3Å (28 mg) versetzt und dann für 48 h bei Raumtemperatur gerührt. Das Reaktionsgemisch wurde über Kieselgur abgesaugt und im Vakuum zur Trockne eingeengt. Der Rückstand wurde in Ethylacetat aufgenommen, mit 1 N Salzsäure gewaschen, die organische Phase über Natriumsulfat getrocknet und im Vakuum zur Trockne eingeengt. Der Rückstand wurde an einer präparativen HPLC mit Wasser/Acetonitril als Laufmittel chromatographiert (Gradient = 40 min von 10% Acetonitril in Wasser auf 100% Acetonitril). Es wurden 2,3 mg der Titelverbindung erhalten. HPLC-MS: logP = 2,19; Masse (m/z): 364,1 (M+H)⁺; ¹H-NMR (CD₃CN) 3.85 (s, 3H) 7.14 - 7.16 (m, 1H), 7.21 - 7.23 (m, 1H), 7.49 - 7.51 (m, 2H), 7.58 - 7.61 (m, 1H), 7.67 - 7.70 (m, 2H), 7.77 - 7.78 (m, 1H), 9,20 (s, 1H).

### Beispiel 8:

### N-[2-(4-Fluor-2-methoxyphenyl)-2H-tetrazol-5-yl]-2-(trifluormethyl)benzamid (Tabelle 2, Beispiel I-2-2)

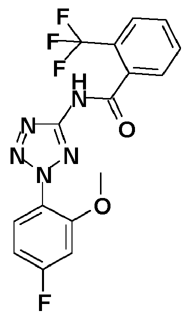

(4-Fluor-2-methoxyphenyl)boronsäure (74 mg) wurde in N,N-Dimethylformamid (1,5 mL) vorgelegt, anschließend mit N-(2H-Tetrazol-5-yl)-2-(trifluoromethyl)benzamid (61 mg), Kupfer(II)acetat (59 mg), Pyridin (35 mg) und Molekularsieb 3Å (28 mg) versetzt und dann für 48 h bei Raumtemperatur gerührt. Das Reaktionsgemisch wurde auf Kieselgel adsorbiert und mit Wasser / 1% Ameisensäure / Acetonitril als Laufmittel zunächst säulenchromatographisch (Gradient = 15 min von 95 % Wasser auf 100 % Acetonitril) gereinigt und dann erneut an einer präparativen HPLC mit Wasser 1% Ameisensäure/Acetonitril als Laufmittel chromatographiert (Gradient = 40 min von 10% Acetonitril in Wasser / 1% Ameisensäure auf 100% Acetonitril). Es wurden 1,5 mg der Titelverbindung erhalten. HPLC-MS: logP = 2,35; Masse (m/z): 382,2 (M+H)⁺; ¹H-NMR (CD₃CN) 3.82 (s, 3H) 6.89 - 6.92 (m, 1H), 7.01 - 7.03 (m, 1H), 7.52 - 7.54 (m, 2H), 7.67 - 7.72 (m, 2H), 7.78 - 7.79(m, 1H), 9.23 (s, 1H)

Die in den Tabellen 1 und 2 beschriebenen Verbindungen der Formeln (I-1) und (I-2) sind ebenfalls bevorzugte Verbindungen, die gemäß oder analog zu den oben beschriebenen Beispielen erhalten wurden.

**Tabelle 1**

| | | | | | |
|---|---|---|---|---|---|
| 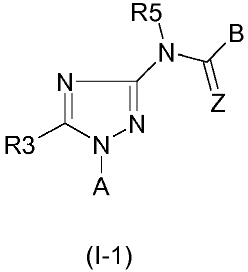 | | | | | |

| **Verbindung** Nr. | **A** | **B** | **R3** | **R5** | **Z** |
|---|---|---|---|---|---|
| I-1-1 | 2-Fluorphenyl | 2-Chlorphenyl | H | H | O |
| I-1-2 | 2-Fluorphenyl | 2-(Trifluormethyl)phenyl | H | H | O |
| I-1-3 | 2-Chlorphenyl | 2-(Trifluormethyl)phenyl | H | H | O |
| I-1-4 | 2-Chlorphenyl | 2-Bromphenyl | H | H | O |
| I-1-5 | 2-Chlorphenyl | 2-Chlorphenyl | H | H | O |
| I-1-6 | 2-Chlorphenyl | 2,6-Difluorphenyl | H | H | O |
| I-1-7 | 2-(Trifluormethyl)phenyl | 2-Chlorphenyl | H | H | O |
| I-1-8 | 2-(Trifluormethyl)phenyl | 2-(Trifluormethyl)phenyl | H | H | O |
| I-1-9 | 2-(Trifluormethoxy)phenyl | 2-Chlorphenyl | H | H | O |
| I-1-10 | 2-(Trifluormethoxy)phenyl | 2-(Trifluormethyl)phenyl | H | H | O |
| I-1-11 | 2,6-Difluorphenyl | 2-(Trifluormethyl)phenyl | H | H | O |
| I-1-12 | 2,6-Difluorphenyl | 2-Bromphenyl | H | H | O |
| I-1-13 | 2,6-Difluorphenyl | 2-Chlorphenyl | H | H | O |
| I-1-14 | 2,6-Difluorphenyl | 2-Methylphenyl | H | H | O |
| I-1-15 | 2,6-Difluorphenyl | 2-Ethylphenyl | H | H | O |
| I-1-16 | 2,6-Difluorphenyl | 2-Methoxyphenyl | H | H | O |
| I-1-17 | 2,6-Difluorphenyl | 2-(Trifluormethoxy)phenyl | H | H | O |
| I-1-18 | 2,6-Difluorphenyl | 2-(Difluormethyl)phenyl | H | H | O |
| I-1-19 | 2,6-Difluorphenyl | 5-Fluor-2-(trifluormethyl)phenyl | H | H | O |
| I-1-20 | 2,6-Difluorphenyl | 2-Fluor-6-(trifluormethyl)phenyl | H | H | O |
| I-1-21 | 2,6-Difluorphenyl | 2-Iodphenyl | H | H | O |
| I-1-22 | 2,6-Difluorphenyl | 2-Nitrophenyl | H | H | O |
| I-1-23 | 2,6-Difluorphenyl | 2-Chlorpyridin-3-yl | H | H | O |
| I-1-24 | 2,6-Difluorphenyl | 3-(Trifluormethyl)pyrazin-2-yl | H | H | O |
| I-1-26 | 2-Fluor-6-(trifluormethyl)phenyl | 2-(Trifluormethyl)phenyl | H | H | O |
| I-1-27 | 2-Fluor-6-(trifluormethyl)phenyl | 2-Chlorphenyl | H | H | O |
| I-1-28 | 2-Fluor-6-(trifluormethyl)phenyl | 2-Iodphenyl | H | H | O |
| I-1-29 | 2-Fluor-6-(trifluormethyl)phenyl | 2- Ethylphenyl | H | H | O |
| I-1-30 | 2-Brom-6-fluorphenyl | 2-(Trifluormethyl)phenyl | H | H | O |
| I-1-31 | 2-Brom-6-fluorphenyl | 2-Chlorphenyl | H | H | O |
| I-1-32 | 2-Brom-6-fluorphenyl | 2-Iodphenyl | H | H | O |
| I-1-33 | 2-Chlor-6-fluorphenyl | 2-(Trifluormethyl)phenyl | H | H | O |
| I-1-34 | 2-Chlor-6-fluorphenyl | 2-(Trifluormethoxy)phenyl | H | H | O |
| I-1-35 | 2-Chlor-6-fluorphenyl | 2-Iodphenyl | H | H | O |
| I-1-36 | 2-Chlor-6-fluorphenyl | 2-Chlorphenyl | H | H | O |
| I-1-37 | 2-Fluor-6-methylphenyl | 2-(Trifluormethyl)phenyl | H | H | O |
| I-1-38 | 2-Fluor-6-methylphenyl | 2-Chlorphenyl | H | H | O |
| I-1-39 | 2-Fluor-6-methylphenyl | 2-Iodphenyl | H | H | O |
| I-1-40 | 2-Chlor-6-methylphenyl | 2-(Trifluormethyl)phenyl | H | H | O |
| I-1-41 | 2-Chlor-6-methylphenyl | 2-Chlorphenyl | H | H | O |
| I-1-42 | 2-Chlor-6-ethylphenyl | 2-(Trifluormethyl)phenyl | H | H | O |
| I-1-43 | 2-Chlor-6-ethylphenyl | 2-Chlorphenyl | H | H | O |
| I-1-44 | 2,6-Dichlorphenyl | 2-(Trifluormethyl)phenyl | H | H | O |
| I-1-45 | 2,6-Dichlorphenyl | 2-Bromphenyl | H | H | O |
| I-1-46 | 2,6-Dichlorphenyl | 2-Chlorphenyl | H | H | O |
| I-1-47 | 2,6-Dimethylphenyl | 2-Chlorphenyl | H | H | O |
| I-1-48 | 2,6-Dimethylphenyl | 2-(Trifluormethyl)phenyl | H | H | O |
| I-1-49 | 2-Methoxy-6-methylphenyl | 2-(Trifluormethyl)phenyl | H | H | O |
| I-1-50 | 2-Methoxy-6-methylphenyl | 2-Chlorphenyl | H | H | O |
| I-1-51 | 2,3-Dichlorphenyl | 2-(Trifluormethyl)phenyl | H | H | O |
| I-1-52 | 2,3-Dichlorophenyl | 2-Chlorphenyl | H | H | O |
| I-1-53 | 2,4-Dichlorophenyl | 2-(Trifluormethyl)phenyl | H | H | O |
| I-1-54 | 2,5-Dichlorophenyl | 2-(Trifluormethyl)phenyl | H | H | O |
| I-1-55 | 2,5-Dichlorophenyl | 2-Chlorphenyl | H | H | O |
| I-1-57 | 2,3,6-Trifluorphenyl | 2-(Trifluormethyl)phenyl | H | H | O |
| I-1-58 | 2,3,6-Trifluorphenyl | 2-Chlorphenyl | H | H | O |
| I-1-59 | 2,3,6-Trifluorphenyl | 2-Iodphenyl | H | H | O |
| I-1-60 | 2,3,6-Trifluorphenyl | 2-Ethylphenyl | H | H | O |
| I-1-61 | 2,4,6-Trifluorphenyl | 2-(Trifluormethyl)phenyl | H | H | O |
| I-1-62 | 2,4,6-Trifluorphenyl | 2-Chlorphenyl | H | H | O |
| I-1-63 | 2,4,6-Trifluorphenyl | 2-Iodphenyl | H | H | O |
| I-1-64 | 2,4,6-Trifluorphenyl | 2- Ethylphenyl | H | H | O |
| I-1-65 | Pentafluorphenyl | 2-(Trifluormethyl)phenyl | H | H | O |
| I-1-66 | Pentafluorphenyl | 2-Chlorphenyl | H | H | O |
| I-1-67 | Pentafluorphenyl | 2-Iodphenyl | H | H | O |
| I-1-68 | Pentafluorphenyl | 2-Ethylphenyl | H | H | O |
| I-1-70 | 2-Bromphenyl | 2-(Trifluormethyl)phenyl | H | H | O |
| I-1-71 | 2-Bromphenyl | 2-Chlorphenyl | H | H | O |
| I-1-72 | 2-Bromphenyl | 2-Iodophenyl | H | H | O |
| I-1-73 | 2-Bromphenyl | 2-Ethylphenyl | H | H | O |
| I-1-75 | 2-Iodphenyl | 2-(Trifluormethyl)phenyl | H | H | O |
| I-1-76 | 2-Iodphenyl | 2-Chlorphenyl | H | H | O |
| I-1-77 | 2-Iodphenyl | 2-Iodphenyl | H | H | O |
| I-1-78 | 2-Iodphenyl | 2-Ethylphenyl | H | H | O |
| I-1-80 | 2,6-Dibromphenyl | 2-(Trifluormethyl)phenyl | H | H | O |
| I-1-81 | 2,6-Dibromphenyl | 2-Ethylphenyl | H | H | O |
| I-1-83 | 2-Fluor-6-iodphenyl | 2-(Trifluormethyl)phenyl | H | H | O |
| I-1-84 | 2-Fluor-6-iodphenyl | 2-Chlorphenyl | H | H | O |
| I-1-85 | 2-Fluor-6-iodphenyl | 2-Iodphenyl | H | H | O |
| I-1-87 | 2,6-Dibrom-4-fluorphenyl | 2-(Trifluormethyl)phenyl | H | H | O |
| I-1-88 | 2,6-Dibrom-4-fluorphenyl | 2-Chlorphenyl | H | H | O |
| I-1-89 | 3,5-Difluorpyridin-2-yl- | 2-(Trifluormethyl)phenyl | H | H | O |
| I-1-90 | 2,4-Dimethyl-5-(2,2,2-trifluoroethylsulfinyl)phe nyl | 2-(Trifluormethyl)phenyl | H | H | O |
| I-1-91 | 2,6- Difluorphenyl | 3-(Trifluormethyl)-2-pyridyl | H | H | O |
| I-1-92 | 2,3,5,6-Tetrafluorphenyl | 2-Chlorphenyl | H | H | O |
| I-1-93 | 2,3,5,6-Tetrafluorphenyl | 2-(Trifluormethyl)phenyl | H | H | O |
| I-1-94 | 2,6-Difluorphenyl | 2-Iod-3-thienyl | H | H | O |
| I-1-95 | 2-(Trifluormethyl)phenyl | 2-Ethylphenyl | H | H | O |
| I-1-96 | 2,4-Dimethyl-5-(2,2,2-trifluoroethylsulfonyl)phe nyl | 2-(Trifluormethyl)phenyl | H | H | O |
| I-1-97 | 2,3,5-Trifluorphenyl | 2-Iodphenyl | H | H | O |
| I-1-98 | 2,4-Dimethyl-5-(2,2,2-trifluorethylsulfanyl)phen yl | 2-(Trifluormethyl)phenyl | H | H | O |

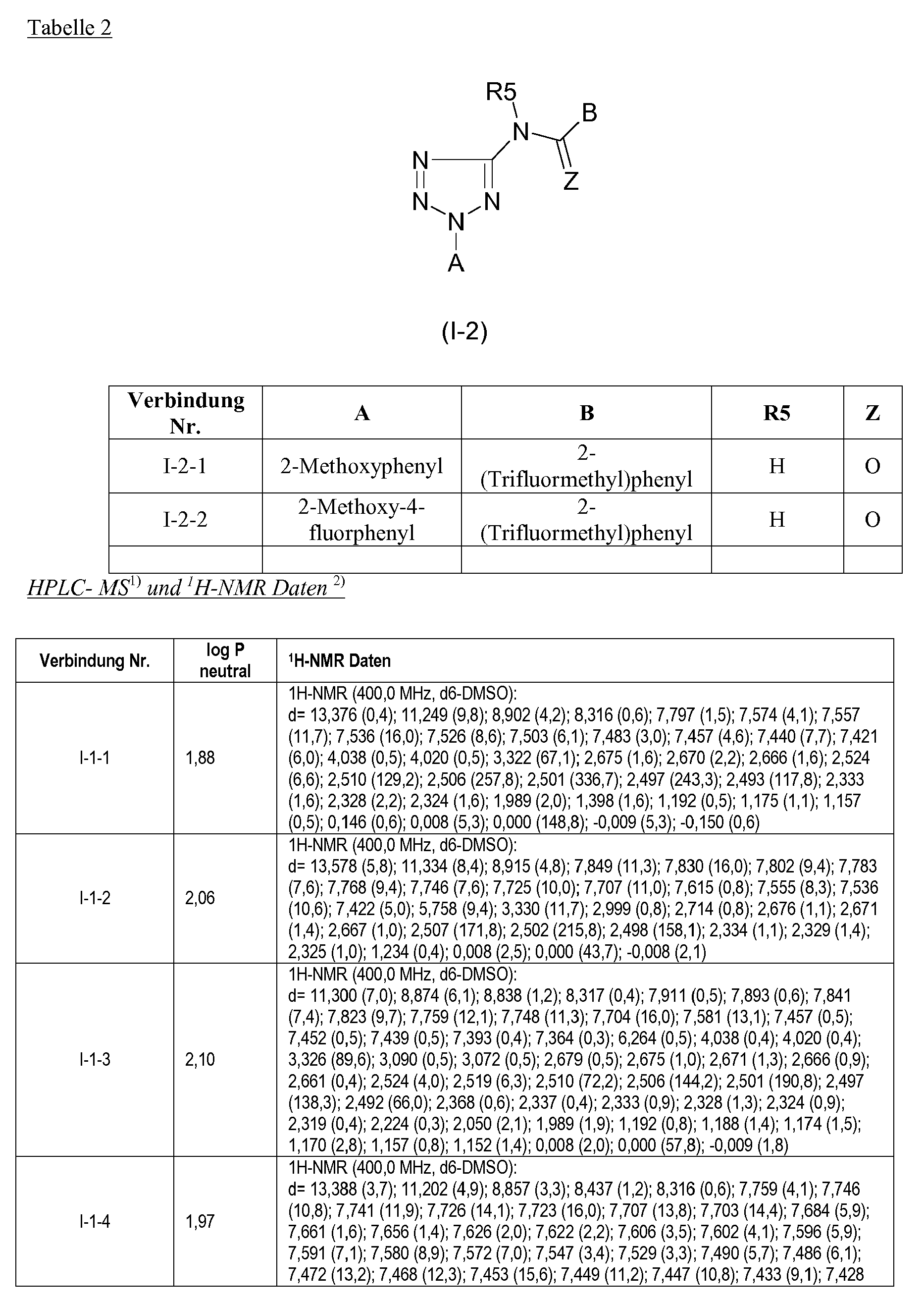

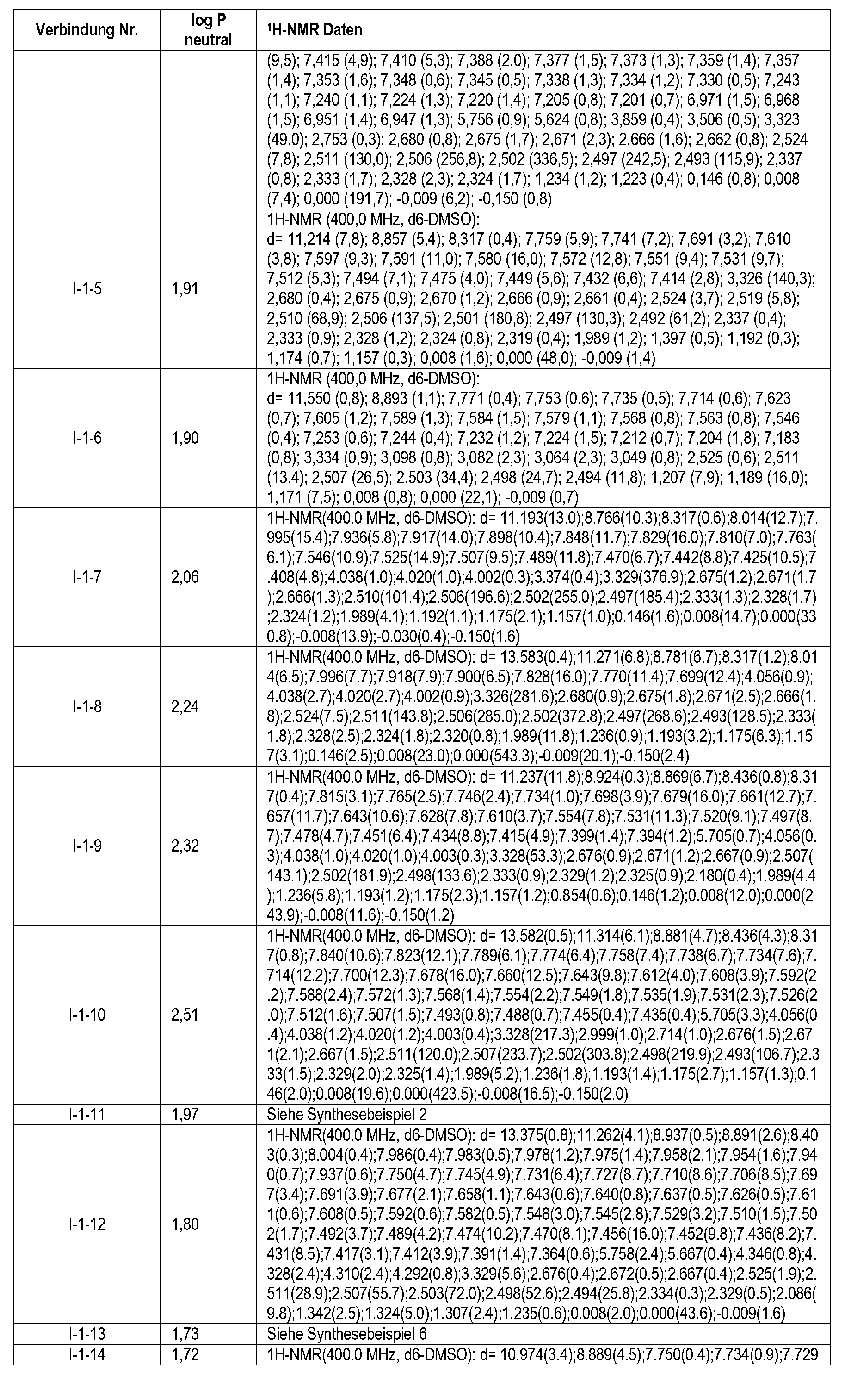

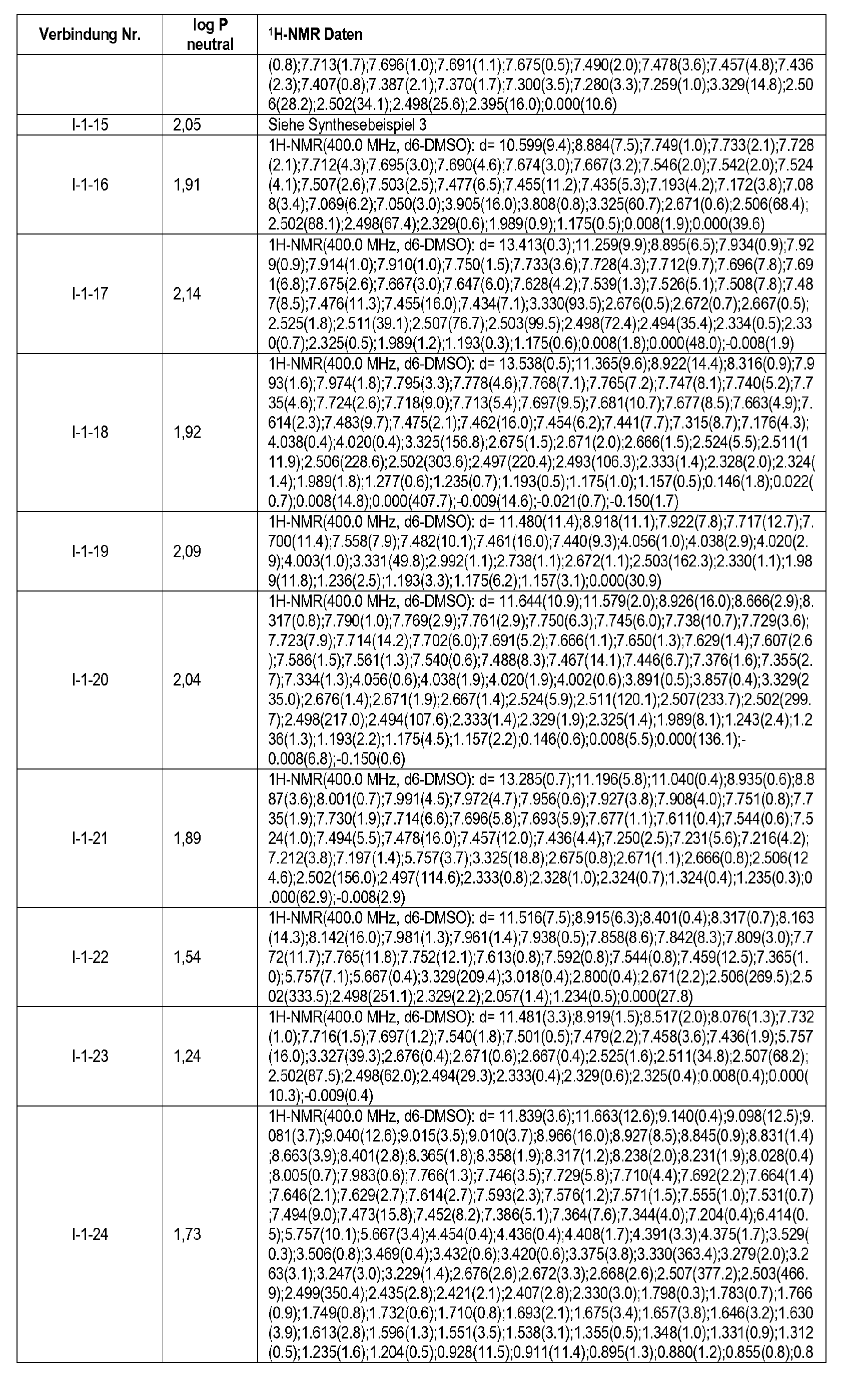

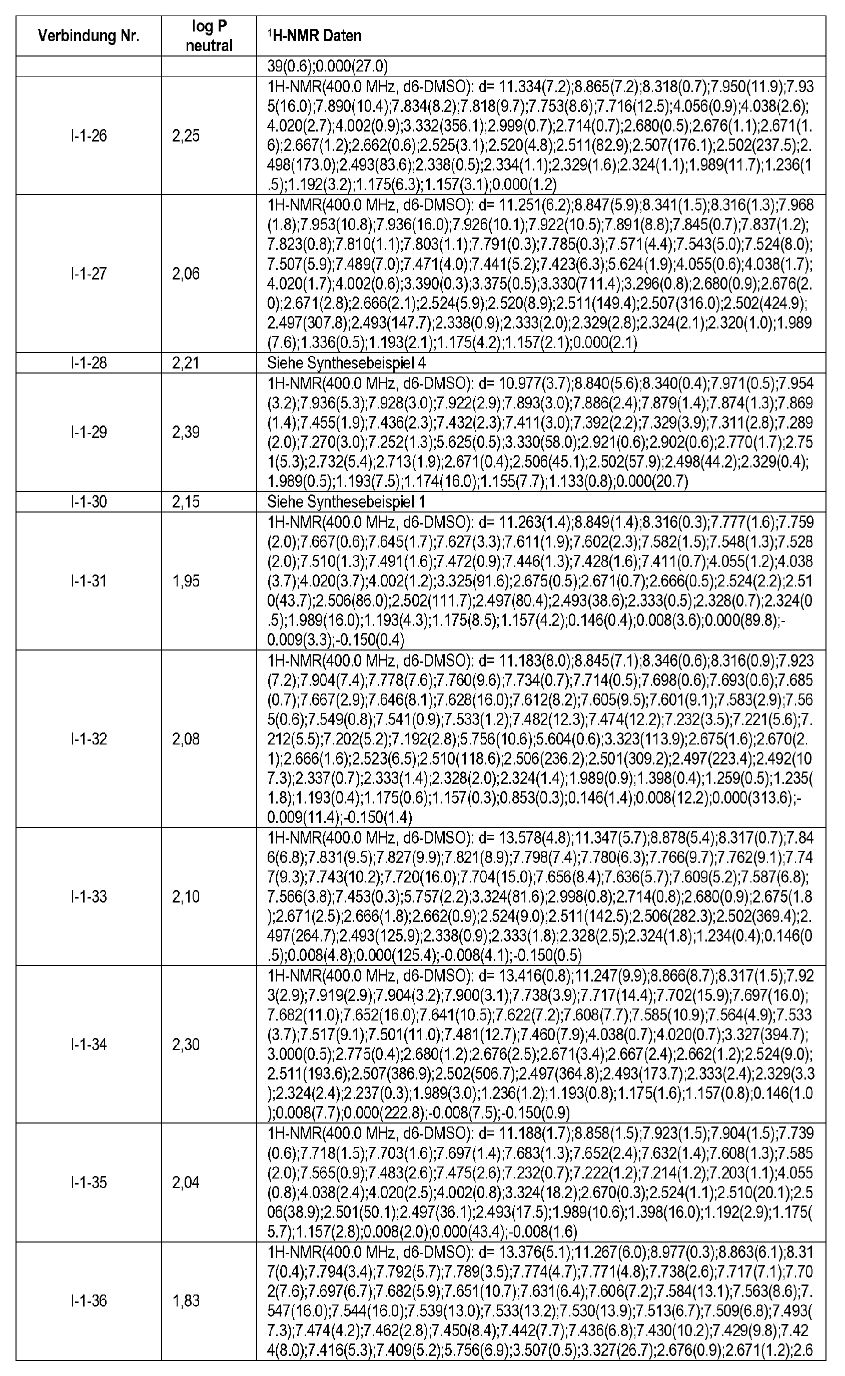

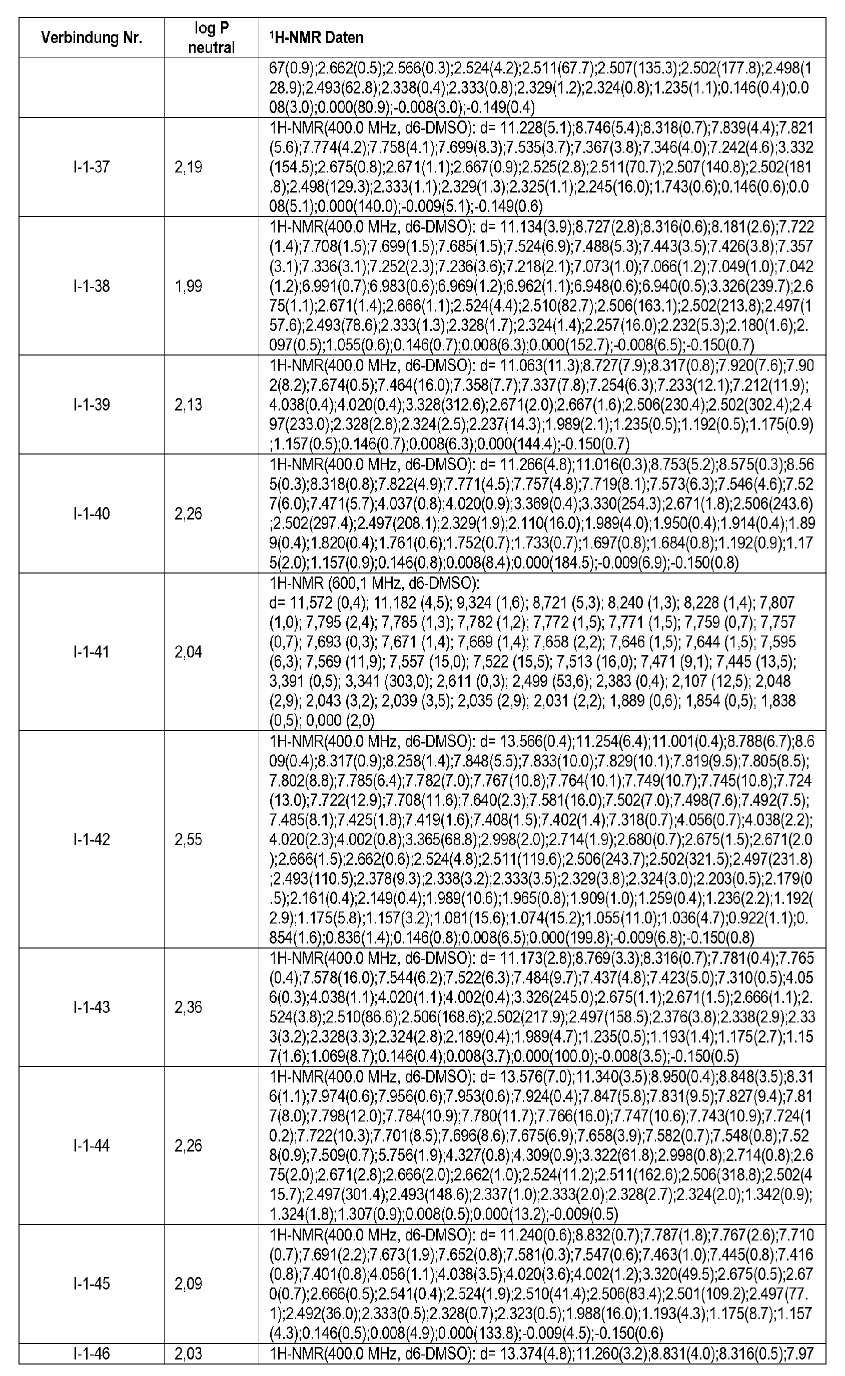

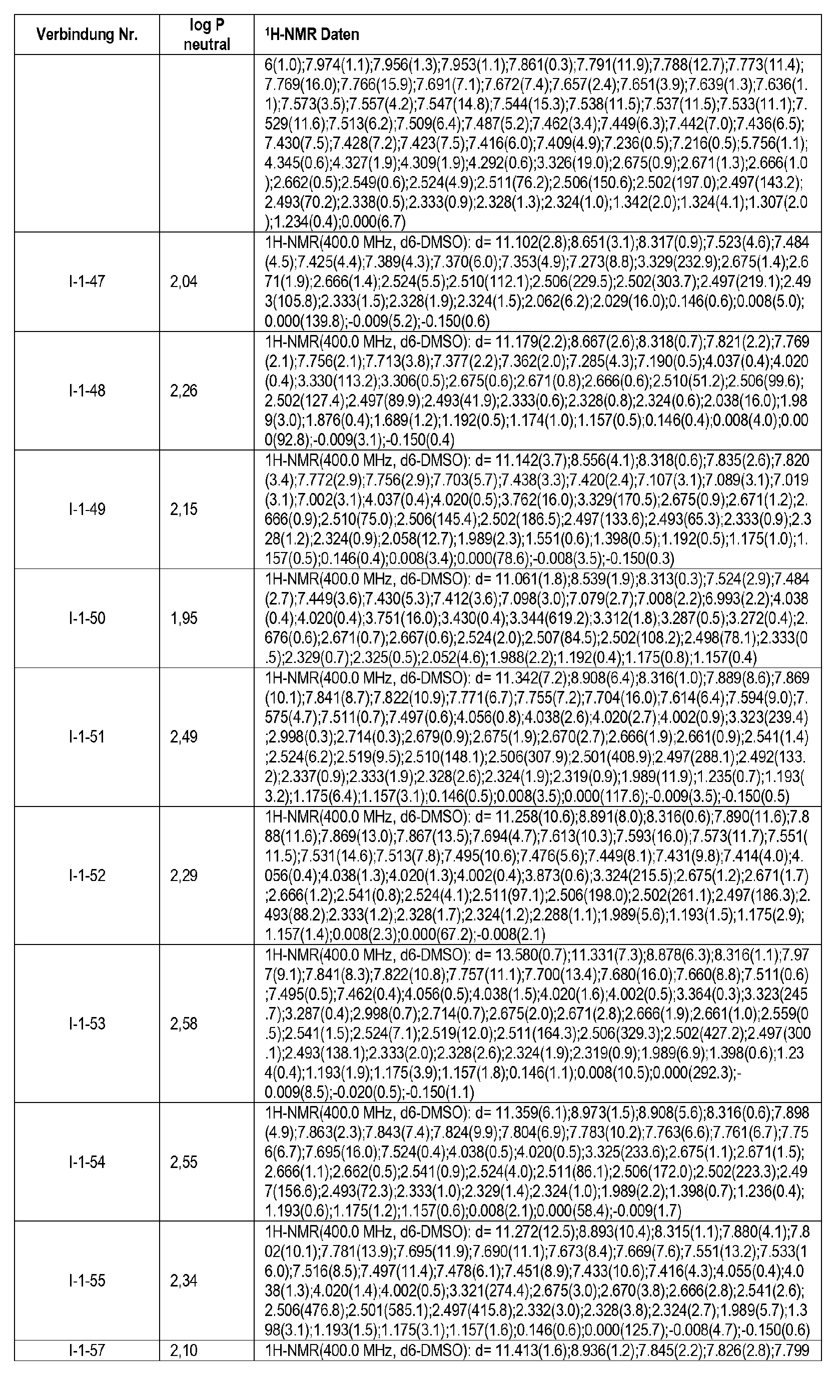

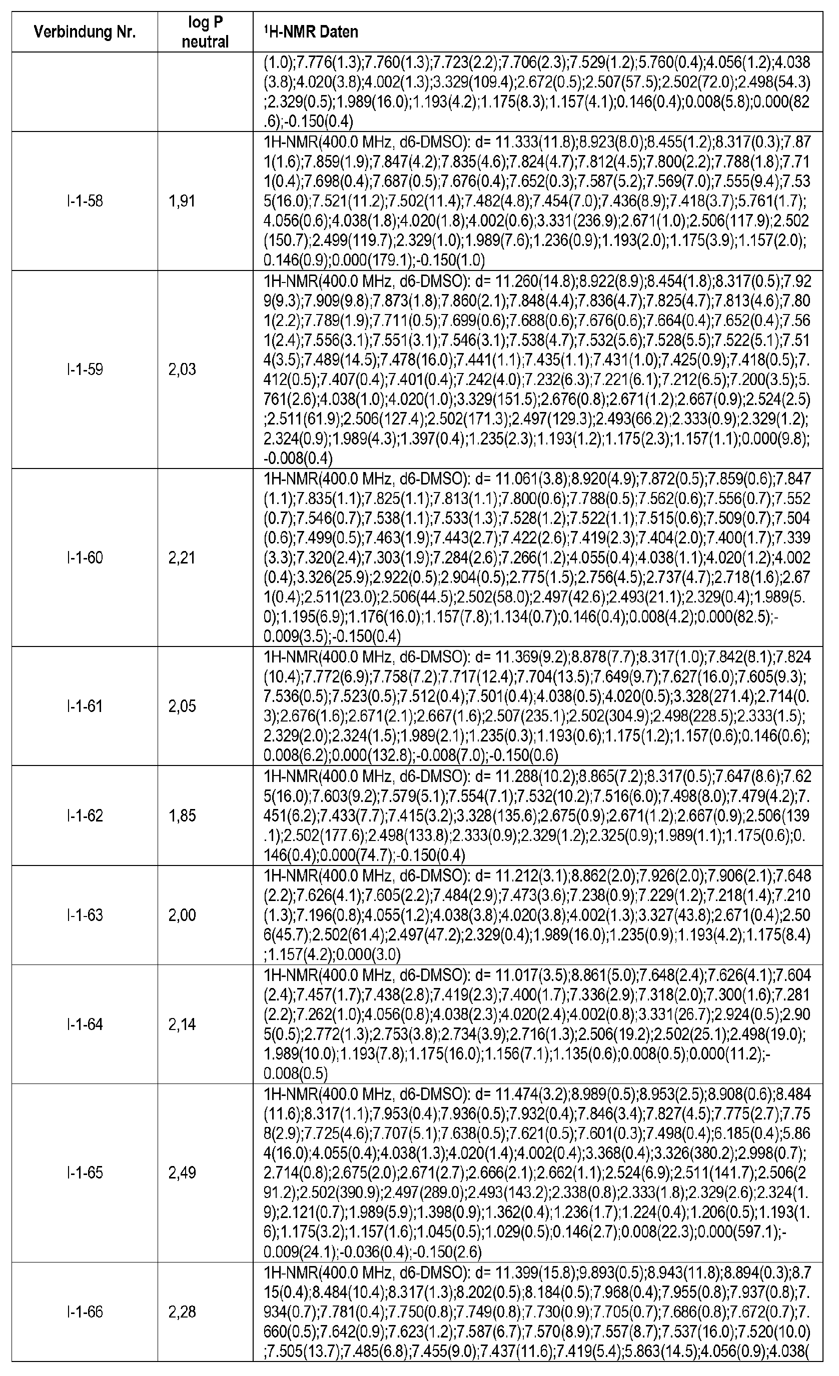

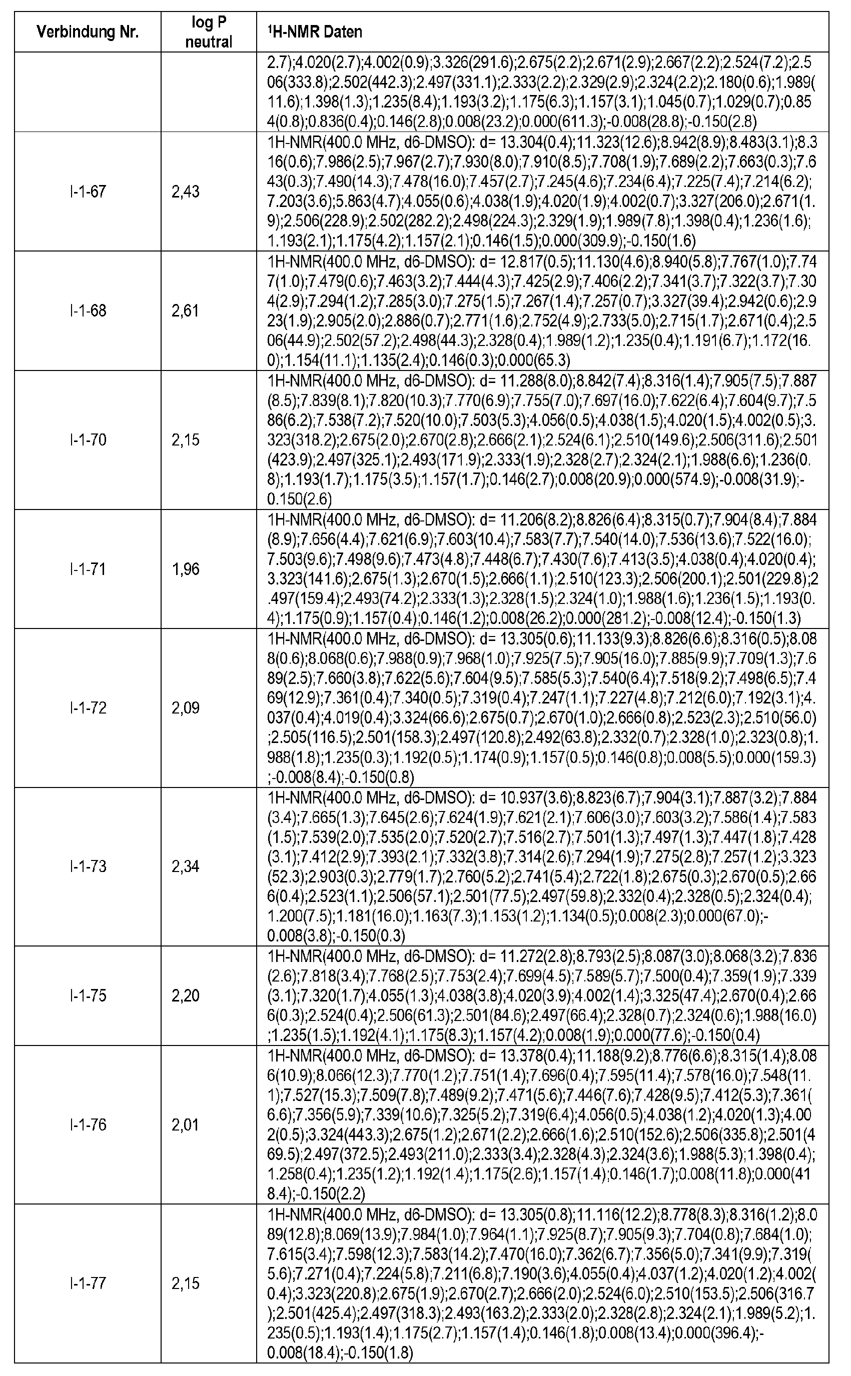

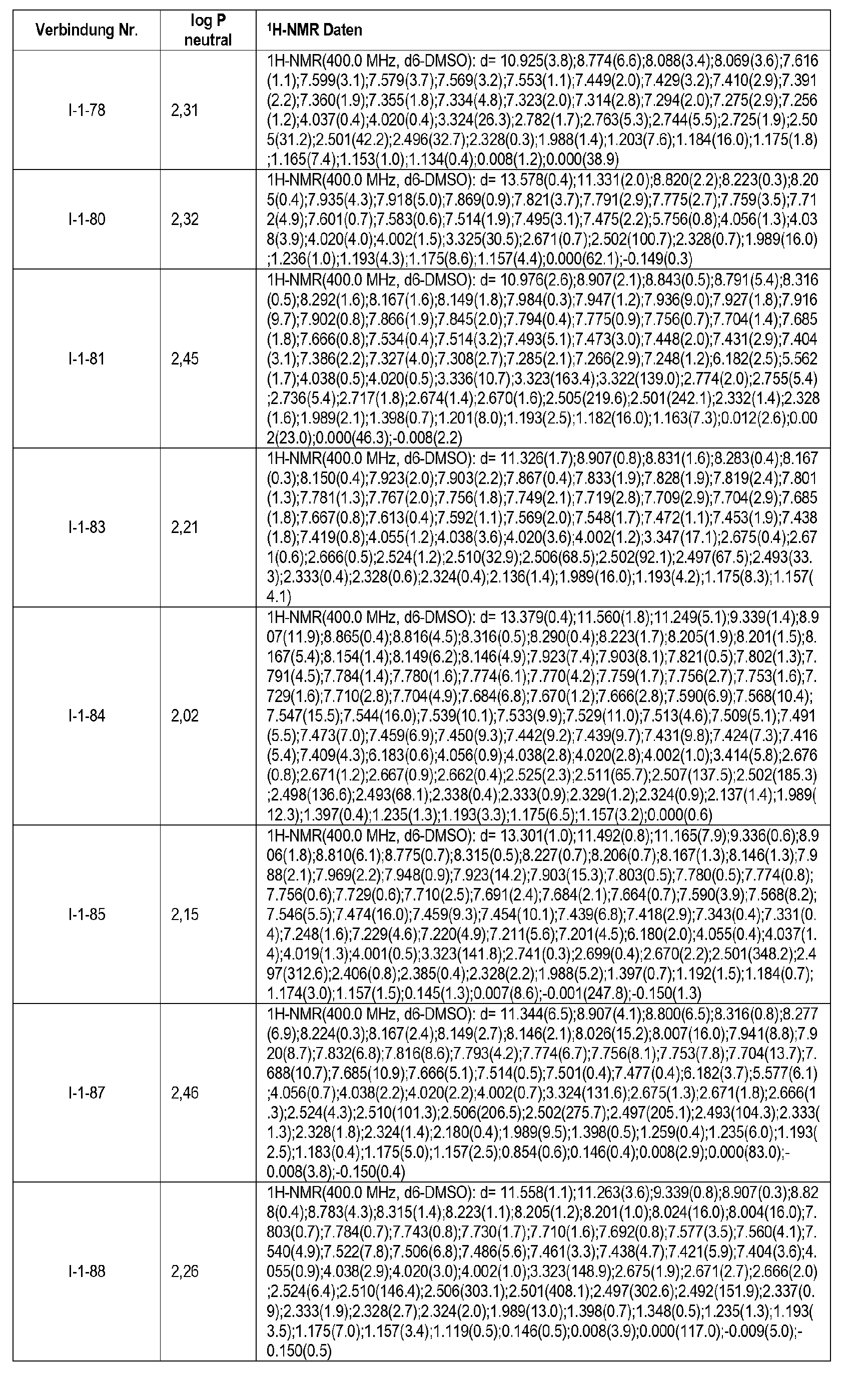

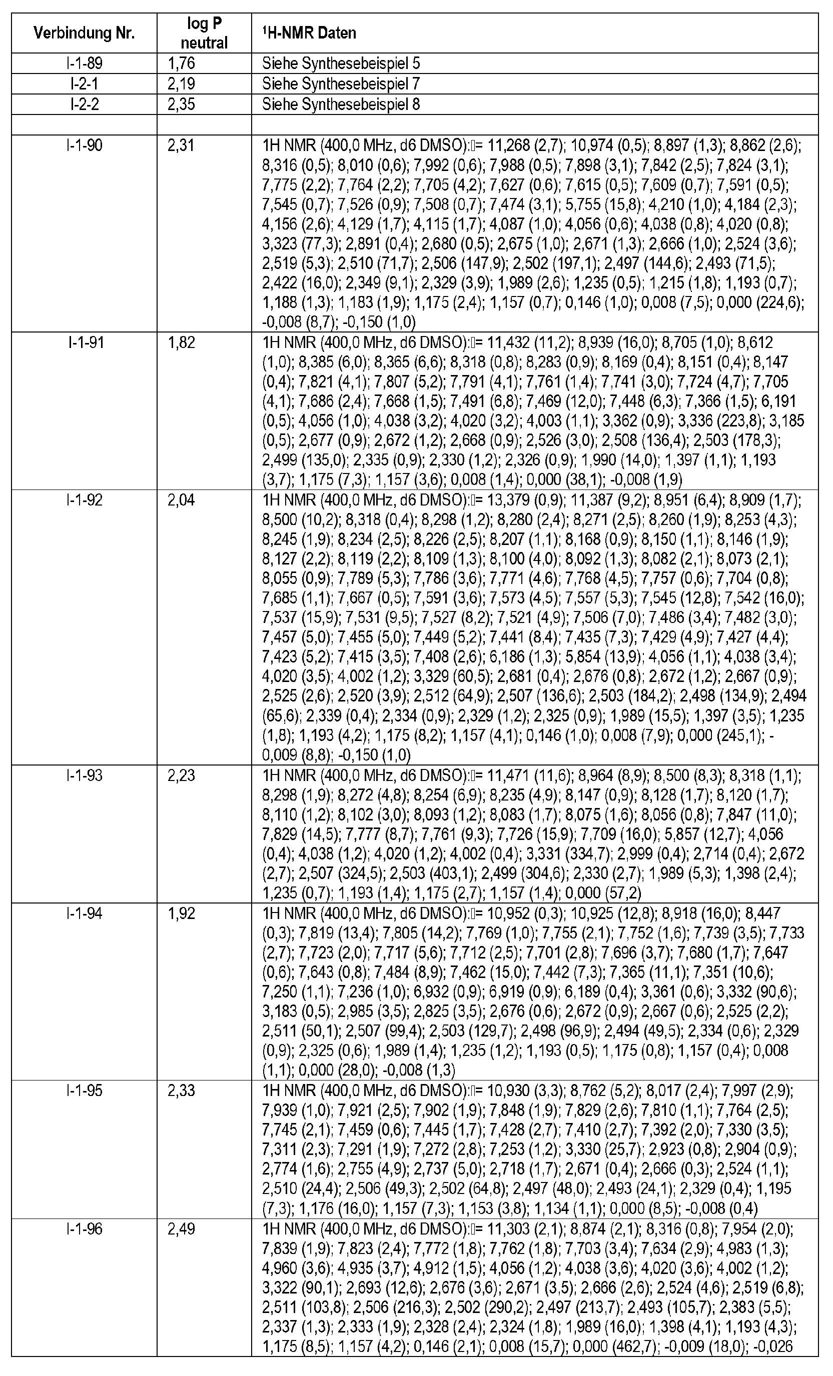

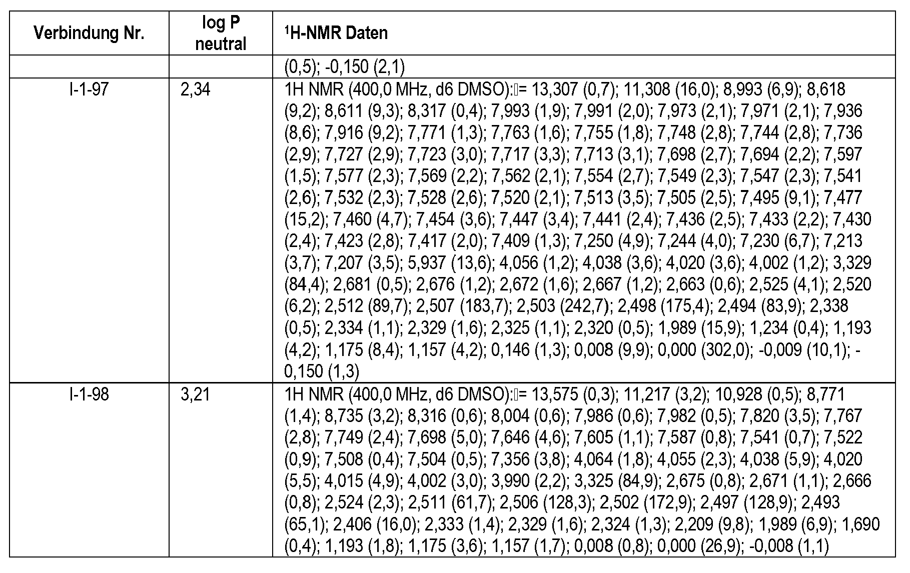

### 1) Methodenbeschreibung zur Bestimmung der logP Werte (Ameisensäure Methode)

Die Bestimmung der in der Tabelle angegebenen logP-Werte erfolgte gemäß EEC-Directive 79/831 Annex V.A8 durch HPLC (High Performance Liquid Chromatography) an einer Phasenumkehrsäule (C 18). Temperatur: 55°C.

Eluenten für die Bestimmung im sauren Bereich (pH 3,4):
Eluenat A: Acetonitril + 1 ml Ameisensäure/Liter. Eluent B: Wasser + 0,9 ml Ameisensäure/Liter.
Gradient: von 10% Eluent A / 90% Eluent B bis 95% Eluent A / 5% Eluent B in 4,25 min.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen). Die lambda-max-Werte wurden an Hand der UV-Spektren von 200 nm bis 400 nm in den Maxima der chromatographischen Signale ermittelt.

### 2) Messung der NMR-Spektren

Die NMR-Spektren wurden mit einem Bruker Avance 400, ausgestattet mit einem Durchflussprobenkopf (60 µl Volumen), bestimmt. Als Lösungsmittel wurden CD₃CN oder DMSO-D₆ verwendet, wobei als Referenz Tetramethylsilan (0.00 ppm) eingesetzt wurde. In Einzelfällen wurden die NMR Spektren mit einem Bruker Avance II 600 bestimmt. Als Lösungsmittel wurden CD₃CN oder DMSO-D₆ verwendet, wobei als Referenz Tetramethylsilan (0.00 ppm) eingesetzt wurde.

Die Aufstpaltung der Signale wurde wie folgt beschrieben: s (Singulett), d (Duplett), t (Triplett), q (Quartett), quin (Quintett), m (Multiplett).

### NMR- Peak- Listenverfahren

Wenn die 1H-NMR-Daten ausgewählter Beispiele in Form von 1H-NMR-Peaklisten notiert werden, wird zu jedem Signalpeak erst der d-Wert in ppm und dann die Signalintensität in runden Klammern aufgeführt. Die d-Wert - Signalintensitäts- Zahlenpaare von verschiedenen Signalpeaks werden durch Semikolons voneinander getrennt aufgelistet.

Die Peakliste eines Beispieles hat daher die Form:
δ1 (Intensität1); δ2 (Intensität2); ........; δi (Intensitäti); ......; δn (Intensitätn)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D6 und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

### Anwendungsbeispiele

Die folgenden Beispiele zeigen die insektizide, akarizide und nematizide Wirkung der erfindungsgemäßen Verbindungen. Dabei beziehen sich die genannten erfindungsgemäßen Verbindungen auf die in den Tabellen 1 und 2 mit den entsprechenden Referenzzeichen, z. B. I-1-1, aufgeführten Verbindungen:

### Boophilus microplus -Injektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf noch etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigten z.B. die folgenden Verbindungen der Herstellungsbeispiele eine Wirkung von 100% bei einer Aufwandmenge von 20 µg/Tier: I-1-10, I-1-11, I-1-24, I-1-30, I-1-33, I-1-59, I-1-65

Bei diesem Test zeigten z.B. die folgenden Verbindungen der Herstellungsbeispiele eine Wirkung von 90% bei einer Aufwandmenge von 20 µg/Tier: I-1-44, I-1-57

Bei diesem Test zeigten z.B. die folgenden Verbindungen der Herstellungsbeispiele eine Wirkung von 80% bei einer Aufwandmenge von 20 µg/Tier: I-1-18

### Cooperia curticei - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration.

Gefäße mit der Wirkstoffzubereitung der gewünschten Konzentration werden mit ca. 40 Nematodenlarven *(Cooperia curticei)* besetzt.

Nach 5 Tagen wird die Abtötung in % bestimmt. Dabei bedeutet 100 %, dass alle Larven abgetötet wurden; 0 % bedeutet, dass keine der Larven abgetötet wurde.

Bei diesem Test zeigten z.B. die folgenden Verbindungen der Herstellungsbeispiele eine Wirkung von 100% bei einer Aufwandmenge von 20 ppm: I-1-20, I-1-21, I-1-33, I-1-57, I-1-63

Bei diesem Test zeigten z.B. die folgenden Verbindungen der Herstellungsbeispiele eine Wirkung von 90% bei einer Aufwandmenge von 20 ppm: I-1-26, I-1-59

Bei diesem Test zeigten z.B. die folgenden Verbindungen der Herstellungsbeispiele eine Wirkung von 80% bei einer Aufwandmenge von 20 ppm: I-1-28, I-1-30, I-1-61, I-1-70, I-1-83, I-1-85

### Haemonchus contortus - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration.

Gefäße mit der Wirkstoffzubereitung der gewünschten Konzentration werden mit ca. 40 Larven des Roten Magenwurmes *(Haemonchus contortus)* besetzt.

Nach 5 Tagen wird die Abtötung in % bestimmt. Dabei bedeutet 100 %, dass alle Larven abgetötet wurden; 0 % bedeutet, dass keine der Larven abgetötet wurde.

Bei diesem Test zeigten z.B. die folgenden Verbindungen der Herstellungsbeispiele eine Wirkung von 80% bei einer Aufwandmenge von 20 ppm: 1-1-21

### Lucilia cuprina - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege *(Lucilia cuprina)* werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigten z.B. die folgenden Verbindungen der Herstellungsbeispiele eine Wirkung von 100% bei einer Aufwandmenge von 100 ppm: I-1-11, I-1-24

### Musca domestica-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica*) besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigten z.B. die folgenden Verbindungen der Herstellungsbeispiele eine Wirkung von 80% bei einer Aufwandmenge von 100 ppm: I-1-11, I-1-24

### Meloidogyne incognita- Test (MELGIN)

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigten z. B. die folgenden Verbindungen der Herstellungsbeispiele Wirkung von 100% bei einer Aufwandmenge von 20 ppm: I-1-8, I-1-10, I-1-13, I-1-14, I-1-15, I-1-18, I-1-19, I-1-20, I-1-21, I-1-24, I-1-26, I-1-28, I-1-29, I-1-30, I-1-31, I-1-32, I-1-33, I-1-35, I-1-44, I-1-45, I-1-57, I-1-58, I-1-59, I-1-60, I-1-61, I-1-62, I-1-63, I-1-64, I-1-80, I-1-83, I-1-85, I-1-89, I-1-91, I-1-93

Bei diesem Test zeigten z. B. die folgenden Verbindungen der Herstellungsbeispiele Wirkung von 90% bei einer Aufwandmenge von 20 ppm: I-1-4, 1-1-11, 1-1-12, I-1-22, 1-1-27, 1-1-40, I-1-42, 1-1-46, I-1-67, I-1-70, I-1-75, I-1-87, I-1-92

### Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 90% bei einer Aufwandmenge von 500 g/ha: 1-1-72

### Phaedon cochleariae - Sprühtest (PHAECO)

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele Wirkung von 100% bei einer Aufwandmenge von 500g /ha: I-1-33, I-1-91

Bei diesem Test zeigten z. B. die folgenden Verbindungen der Herstellungsbeispiele Wirkung von 83% bei einer Aufwandmenge von 500g /ha: I-1-18

### Tetranychus urticae - Sprühtest, OP-resistent

| | |
|---|---|
| Lösungsmittel: | 78,0 GewichtsteileAceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele eine Wirkung von 95% bei einer Aufwandmenge von 500g/ha: I-1-90

## Patentansprüche

1. Verbindungen der Formel (I) 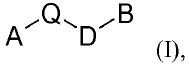 worin
A für einen Rest aus der Reihe 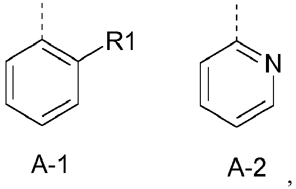 steht, in dem die gestrichelte Linie die Bindung zu Q bedeutet und wobei A weiterhin m Substituenten R2 trägt,
Q für einen Rest aus der Reihe 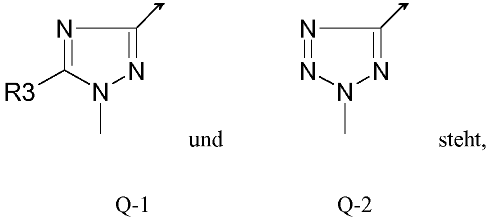 worin der Stickstoff mit dem Ring A verknüpft ist und der Pfeil jeweils die Bindung zu D bedeutet,
D für den Rest der Formel 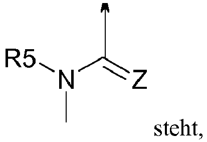 worin der Stickstoff mit Q verbunden ist und der Pfeil die Bindung zu B bedeutet,
B für einen Rest aus der Reihe 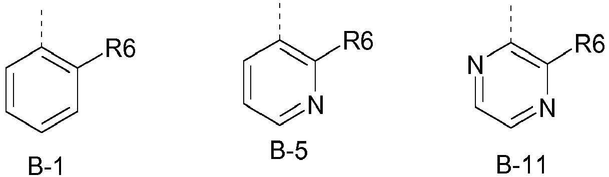 steht, worin die gestrichelte Linie die Bindung zu D bedeutet und wobei B weiterhin n Substituenten R7 trägt,
Z für Sauerstoff steht,
R1 für einen Rest aus der Reihe Halogen, C₁-C₄-Alkyl, C₁-C₄-Halogenalkyl, C₁-C₄-Alkoxy und C₁-C₄-Halogenalkoxy steht,
R2 für einen Rest aus der Reihe Halogen, C₁-C₄-Alkyl, C₁-C₄-Halogenalkyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl und C₁-C₆-Halogenalkylsulfonyl steht,
R3 für Wasserstoff steht,
R5 für Wasserstoff steht,
R6 für einen Rest aus der Reihe Halogen, Nitro, C₁-C₄-Alkyl, C₁-C₄-Halogenalkyl und C₁-C₄-Halogenalkoxy steht,
R7 für Halogen steht,
m für eine Zahl aus der Reihe 0, 1, 2, 3 und 4 steht, wobei für m > 1 die Reste R2 gleich oder verschieden sein können, und
n für eine Zahl aus der Reihe 0 und 1 steht.

2. Verbindung der Formel (I) gemäß Anspruch 1, worin Q für Q-1 steht und A und B die in der folgenden Tabelle angegebenen Bedeutungen haben
| **Verbindung Nr.** | **A** | **B** |
|---|---|---|
| I-1-11 | 2,6-Difluorphenyl | 2-(Trifluormethyl)phenyl |
| I-1-20 | 2,6-Difluorphenyl | 2-Fluor-6-(trifluormethyl)phenyl |
| I-1-21 | 2,6-Difluorphenyl | 2-Iodphenyl |
| I-1-26 | 2-Fluor-6-(trifluormethyl)phenyl | 2-(Trifluormethyl)phenyl |
| I-1-28 | 2-Fluor-6-(trifluormethyl)phenyl | 2-Iodphenyl |
| I-1-30 | 2-Brom-6-fluorphenyl | 2-(Trifluormethyl)phenyl |
| I-1-33 | 2-Chlor-6-fluorphenyl | 2-(Trifluormethyl)phenyl |
| I-1-44 | 2,6-Dichlorphenyl | 2-(Trifluormethyl)phenyl |
| I-1-57 | 2,3,6-Trifluorphenyl | 2-(Trifluormethyl)phenyl |
| I-1-61 | 2,4,6-Trifluorphenyl | 2-(Trifluormethyl)phenyl |
| I-1-93 | 2,3,5,6-Tetrafluorphenyl | 2-(Trifluormethyl)phenyl |

3. Mittel, **gekennzeichnet durch** einen Gehalt von mindestens einer Verbindung der Formel (I) gemäß Anspruch 1 oder 2.

4. Nicht-therapeutische Verfahren zum Bekämpfen von Schädlingen, **dadurch gekennzeichnet, dass** man eine Verbindung der Formel (I) gemäß Anspruch 1 oder 2 oder ein Mittel gemäß Anspruch 3 auf Schädlinge und/oder ihren Lebensraum einwirken lässt.

5. Verbindungen der Formel (IIa-1-a) 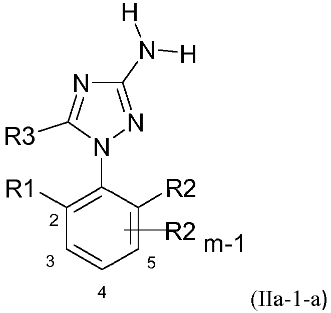 in welchen R1, R2 und R3 die oben genannten Bedeutungen haben und m für eine Zahl aus der Reihe 1, 2 und 3 steht.

6. Verbindungen der Formel (1-9) 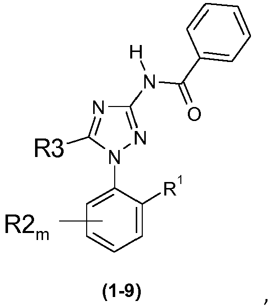 in welchen R1, R2, R3 und m die oben genannten Bedeutungen haben.

## Claims

1. Compounds of formula (I) 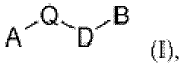 in which
A is a radical from the group of 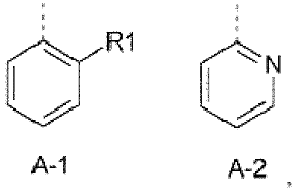 in which the dashed line denotes the bond to Q and wherein A also bears m substituents R2,
Q is a radical from the group of 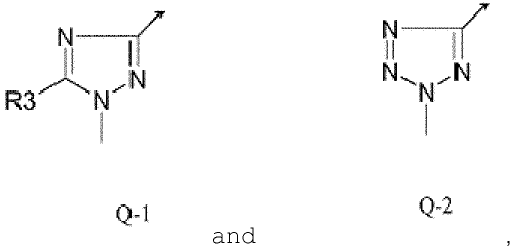 wherein the nitrogen is joined to the ring A and the arrow in each case denotes the bond to D,
D is the radical of formula 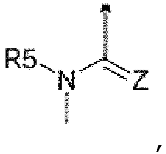 in which the nitrogen is bonded to Q and the arrow denotes the bond to B,
B is a radical from the group 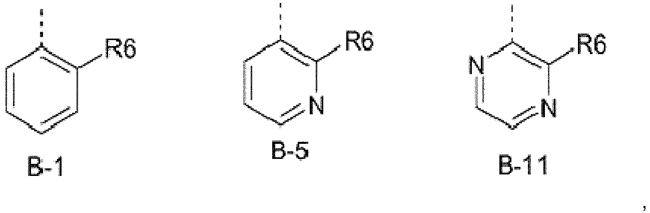 in which the dashed line denotes the bond to D and wherein B also bears n substituents R7,
Z is oxygen,
R1 is a radical from the group of halogen, C₁-C₄-alkyl, C₁-C₄-haloalkyl, C₁-C₄-alkoxy and C₁-C₄-haloalkoxy,
R2 is a radical from the group of halogen, C₁-C₆-haloalkylthio, C₁-C₆-alkylsulfinyl, C₁-C₆-haloalkylsulfinyl, C₁-C₆-alkylsulfonyl and C₁-C₆-haloalkylsulfonyl,
R3 is hydrogen,
R5 is hydrogen,
R6 is a radical from the group of halogen, nitro, C₁-C₄-alkyl, C₁-C₄-haloalkyl, and C₁-C₄-haloalkoxy,
R7 is halogen,
m is a number from the group 0, 1, 2, 3 and 4, wherein, for m>l, the radicals R2 may be identical or different, and
n is a number from the group 0 and 1.

2. Compound of formula (I) according to Claim 1, in which Q is Q-1 and A and B have the meanings given in the following table:
| **Compoun d no.** | **A** | **B** |
|---|---|---|
| I-1-11 | 2,6-difluorophenyl | 2-(trifluoromethyl)pheny l |
| I-1-20 | 2,6-difluorophenyl | 2-fluoro-6-(trifluoromethyl)pheny l |
| I-1-21 | 2,6-difluorophenyl | 2-iodophenyl |
| I-1-26 | 2-fluoro-6-(trifluoromethyl)pheny l | 2-(trifluoromethyl)pheny l |
| I-1-28 | 2-fluoro-6-(trifluoromethyl)pheny l | 2-iodophenyl |
| I-1-30 | 2-bromo-6-fluorophenyl | 2-(trifluoromethyl)pheny l |
| I-1-33 | 2-chloro-6-fluorophenyl | 2-(trifluoromethyl)pheny l |
| I-1-44 | 2,6-dichlorophenyl | 2-(trifluoromethyl)pheny l |
| I-1-57 | 2,3,6-trifluorophenyl | 2-(trifluoromethyl)pheny l |
| I-1-61 | 2,4,6-trifluorophenyl | 2-(trifluoromethyl)pheny l |
| I-1-93 | 2,3,5,6-tetrafluorophenyl | 2-(trifluoromethyl)pheny l |

3. Composition **characterized by** a content of at least one compound of formula (I) according to Claim 1 or 2.

4. Nontherapeutic method for controlling pests, **characterized in that** a compound of formula (I) according to Claim 1 or 2, or a composition according to Claim 3, is allowed to act on pests and/or on their habitat.

5. Compounds of formula (IIa-1-a) 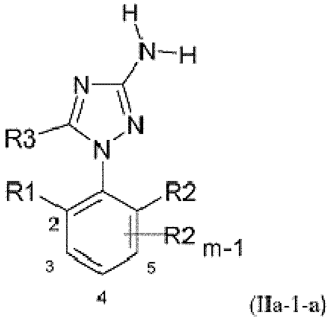 in which R1, R2 and R3 have the abovementioned meanings, and m is a number from the group 1, 2 and 3.

6. Compounds of formula (1-9) 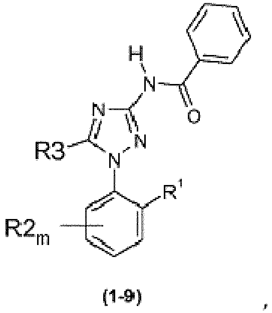 in which R1, R2, R3 and m have the abovementioned meanings.

## Revendications

1. Composés de formule (I) 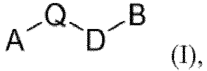 dans laquelle
A représente un radical de la série constituée par 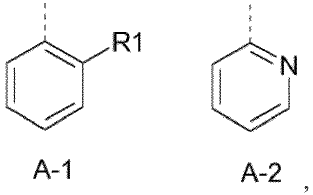
dans lesquels la ligne en pointillés signifie la liaison à Q et A porte en outre m substituants R2,
Q représente un radical de la série constitué par 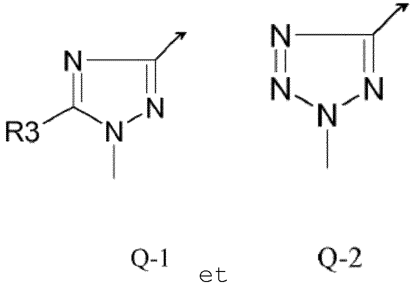 dans lesquels l'azote est relié avec le cycle A et la flèche signifie à chaque fois la liaison à D,
D représente le radical de formule 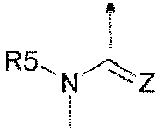
dans lequel l'azote est relié avec Q et la flèche signifie la liaison à B,
B représente un radical de la série constituée par 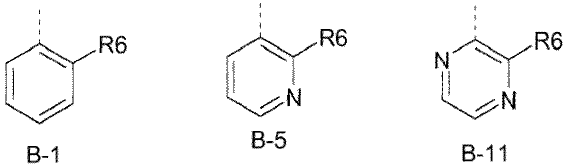
dans lesquels la ligne en pointillés signifie la liaison à D et B porte en outre n substituants R7,
Z représente oxygène,
R1 représente un radical de la série constituée par halogène, alkyle en C₁-C₄, halogénoalkyle en C₁-C₄, alcoxy en C₁-C₄ et halogénoalcoxy en C₁-C₄,
R2 représente un radical de la série constituée par halogène, alkyle en C₁-C₄, halogénoalkyle en C₁-C₄, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆ et halogénoalkylsulfonyle en C₁-C₆,
R3 représente hydrogène,
R5 représente hydrogène,
R6 représente un radical de la série constituée par halogène, nitro, alkyle en C₁-C₄, halogénoalkyle en C₁-C₄ et halogénoalcoxy en C₁-C₄,
R7 représente halogène,
m représente un nombre de la série constituée par 0, 1, 2, 3 et 4, les radicaux R2 pouvant être identiques ou différents lorsque m > 1, et
n représente un nombre de la série constituée par 0 et 1.

2. Composé de formule (I) selon la revendication 1, dans lequel Q représente Q-1 et A et B ont les significations indiquées dans le tableau suivant :
| Composé n° | A | B |
|---|---|---|
| I-1-11 | 2,6-difluorophényle | 2-(trifluorométhyl)phényle |
| I-1-20 | 2,6-difluorophényle | 2-fluoro-6-(trifluorométhyl)phényle |
| I-1-21 | 2,6-difluorophényle | 2-iodophényle |
| I-1-26 | 2-fluoro-6-(trifluorométhyl)phényle | 2-(trifluorométhyl)phényle |
| I-1-28 | 2-fluoro-6-(trifluorométhyl)phényle | 2-iodophényle |
| I-1-30 | 2-bromo-6-fluorophényle | 2-(trifluorométhyl)phényle |
| I-1-33 | 2-chloro-6-fluorophényle | 2-(trifluorométhyl)phényle |
| I-1-44 | 2,6-dichlorophényle | 2-(trifluorométhyl)phényle |
| I-1-57 | 2,3,6-trifluorophényle | 2-(trifluorométhyl)phényle |
| I-1-61 | 2,4,6-trifluorophényle | 2-(trifluorométhyl)phényle |
| I-1-93 | 2,3,5,6-tétrafluorophényle | 2-(trifluorométhyl)phényle |

3. Agent, **caractérisé par** une teneur en au moins un composé de formule (I) selon la revendication 1 ou 2.

4. Procédé non thérapeutique de lutte contre des nuisibles, **caractérisé en ce qu'**un composé de formule (I) selon la revendication 1 ou 2 ou un agent selon la revendication 3 est laissé agir sur des nuisibles et/ou leur habitat.

5. Composés de formule (IIa-1-a) 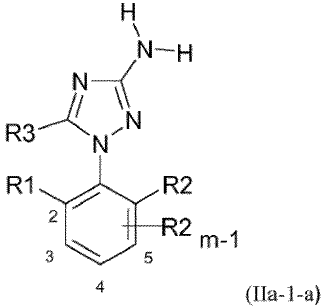 dans laquelle R1, R2 et R3 ont les significations indiquées précédemment et m représente un nombre de la série constituée par 1, 2 et 3.

6. Composés de formule (1-9) 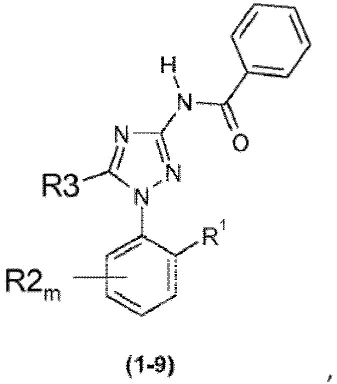 dans laquelle R1, R2, R3 et m ont les significations indiquées précédemment.
